Europäisches Patentamt
European Patent Office
Office européen des brevets

(19)

(11) Publication number: **0 669 334 A1**

(12)

# EUROPEAN PATENT APPLICATION published in accordance with Art. 158(3) EPC

(21) Application number: **93924179.0**

(22) Date of filing: **04.11.93**

(86) International application number: **PCT/JP93/01601**

(87) International publication number: **WO 94/11375 (26.05.94 94/12)**

(51) Int. Cl.[6]: **C07D 487/06**, A61K 31/55

(30) Priority: **11.11.92 JP 300952/92**

(43) Date of publication of application: **30.08.95 Bulletin 95/35**

(84) Designated Contracting States: **AT BE CH DE DK ES FR GB GR IE IT LI LU NL PT SE**

(71) Applicant: **FUJISAWA PHARMACEUTICAL CO., LTD.**
**4-7, Doshomachi 3-chome**
**Chuo-ku**
**Osaka-shi**
**Osaka 541 (JP)**

(72) Inventor: **SATO, Yoshinari**
**7-1-9, Higashihagoromo**
**Takaishi-shi,**
**Osaka 592 (JP)**
Inventor: **ITANI, Hiromichi**
**3-2-47-202, Shioe**
**Amagasaki-shi,**
**Hyogo 661 (JP)**

(74) Representative: **Türk, Dietmar, Dr. rer. nat.**
**Türk, Gille, Hrabal, Leifert**
**Patentanwälte**
**Brucknerstrasse 20**
**D-40593 Düsseldorf (DE)**

(54) **TRICYCLIC COMPOUND.**

(57) A tricyclic compound useful as a medicine, represented by general formula (I), a pharmaceutically acceptable salt thereof, an intermediate therefor, a process for producing the same, and a cholecystokinin antagonist, wherein $R^1$ represents hydrogen, carboxyl, protected carboxyl, carboxy(lower)alkyl, protected carboxy(lower)alkyl, carboxy(lower)alkanoyl, protected carboxy(lower)alkanoyl, carboxy(lower)alkenoyl, protected carboxy(lower)alkenoyl, optionally substitued heterocyclic carbonyl(lower)alkyl, or optionally substituted heterocyclic carbonyl(lower)alkanoyl; $R^2$ represents optionally substituted aryl; and $R^3$ represents acyl.

EP 0 669 334 A1

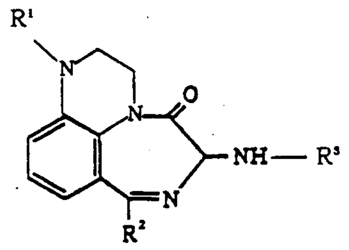

〔 I 〕

Field of the Invention

The present invention relates to a novel tricyclic compound having a cholecystokinin (CCK) antagonism which compound is used in the field of medical treatment.

Background of the Invention

Several tricyclic compounds are known as described in, for example, WO 92/03438.

Disclosure of the Invention

The present invention relates to a novel tricyclic compound or a pharmaceutically acceptable salt thereof.

More specifically, the present invention relates to a novel tricyclic compound or a pharmaceutically acceptable salt thereof which is a cholecystokinin (CCK) antagonist and therefore useful as an agent for preventing and / or treating pancreatitis, cholecystis diseases (for example, acute cholecystitis, calculus, etc.), gastroparesis, pancreatic carcinoma, insulinoma, nausea, vomiting, inappetence, pains, disorders associated with intestinal smooth muscle hyperactivity ( for example, irritable bowel syndrome, splincter contraction, etc.), hyperinsulinism, dyspepsia and the like.

The tricyclic compound of the present invention is represented by the following formula (I).

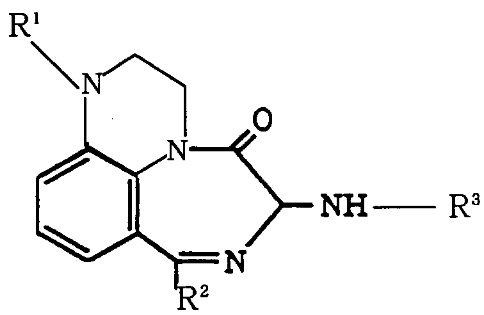

(I)

wherein $R^1$ is hydrogen, carboxy, protected carboxy, carboxy (lower) alkyl, protected carboxy (lower) alkyl, carboxy (lower) alkanoyl, protected carboxy (lower) alkanoyl, carboxy (lower) alkenoyl, protected carboxy (lower) alkenoyl, heterocycliccarbonyl (lower) alkyl which may have suitable substituent (s), or heterocycliccarbonyl (lower) alkanoyl which may have suitable substituent(s),

$R^2$ is aryl which may have suitable substituent(s),

$R^3$ is acyl.

According to the present invention, the new tricyclic compound (I) can be prepared by processes which are illustrated in the following schemes.

Process 1

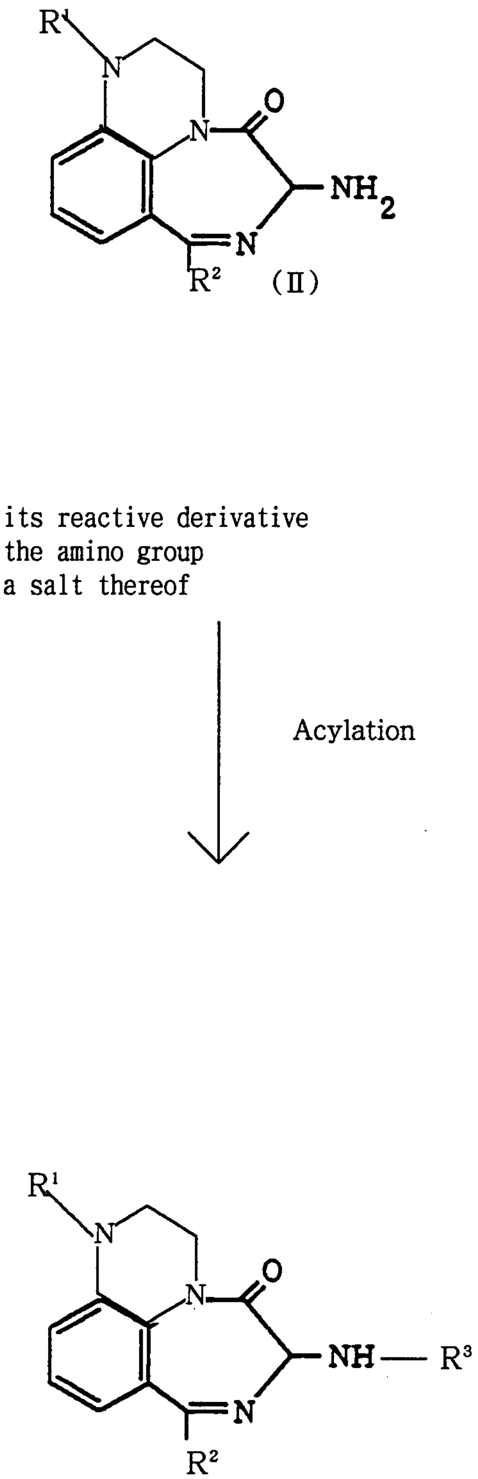


(II)

(I)
or a salt thereof

Process 2

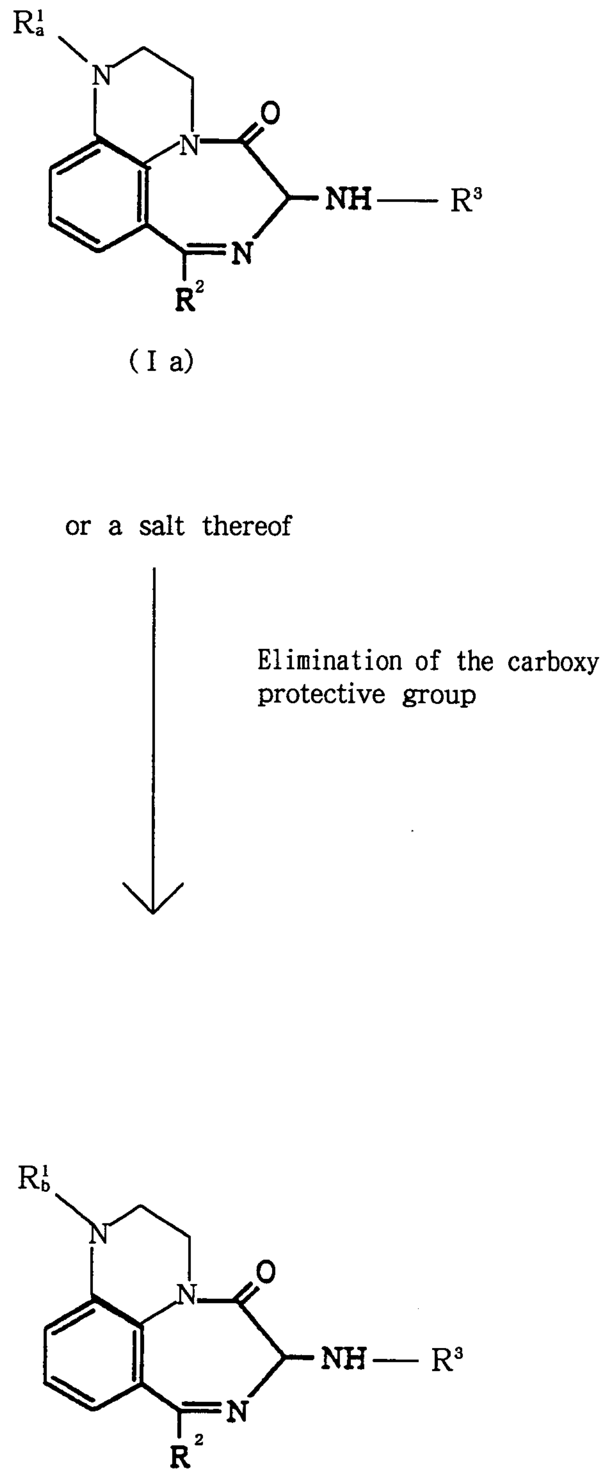


(I a)

or a salt thereof

(I b)

or a salt thereof

Process 3

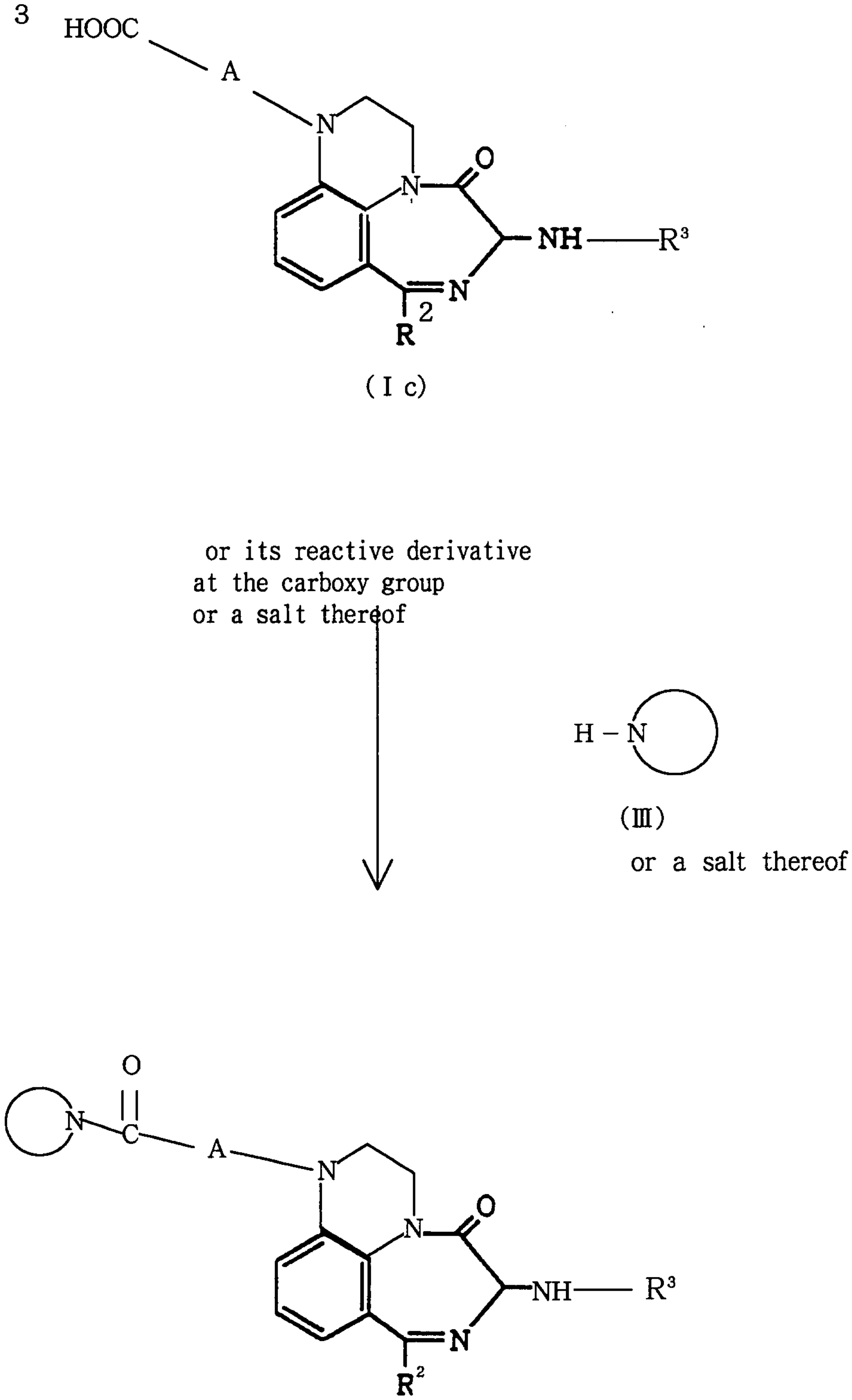

(I c)

or its reactive derivative
at the carboxy group
or a salt thereof

(III)
or a salt thereof

(I d)
or a salt thereof

Process 4

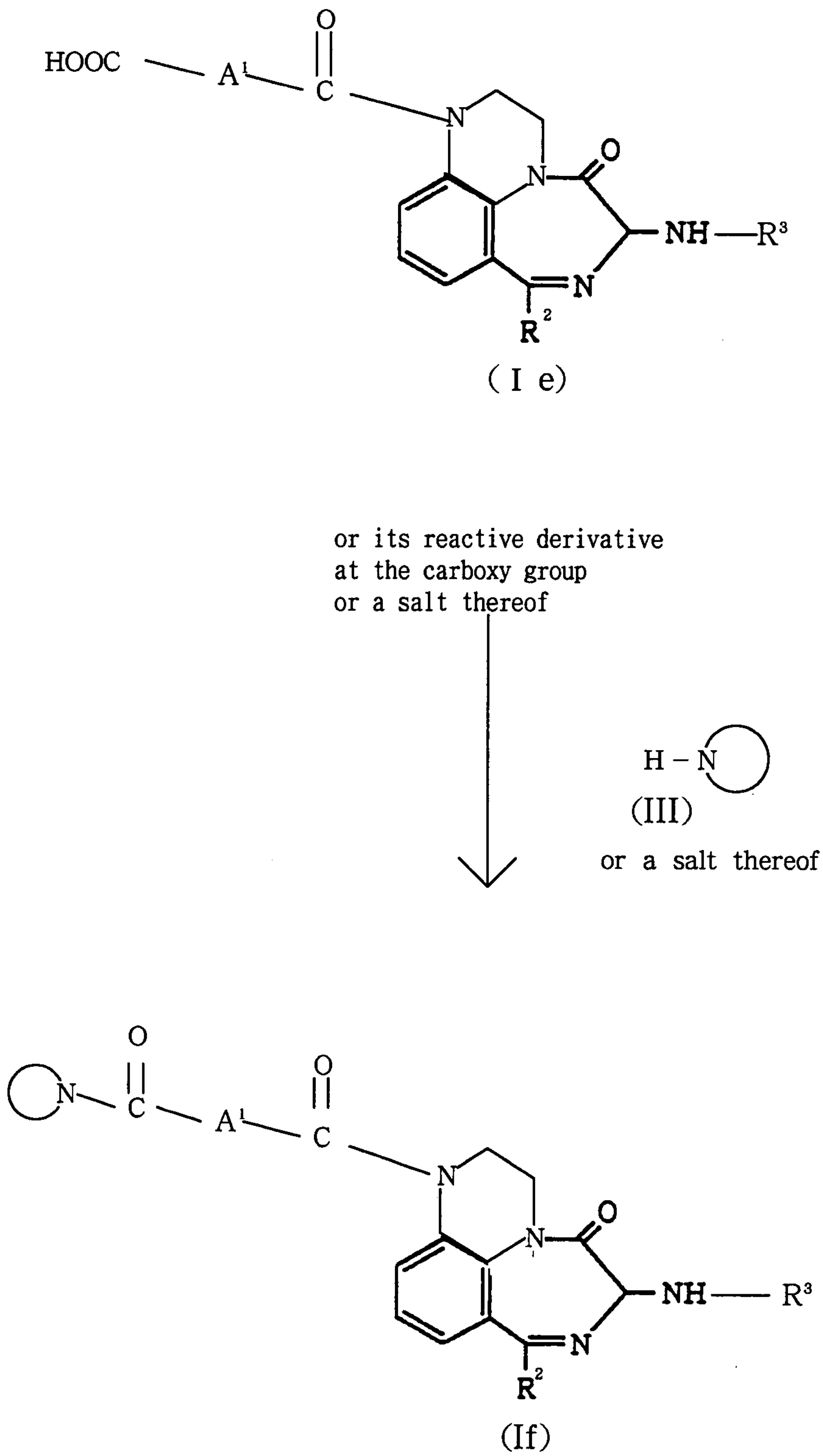


(I e)

(III)

(If)
or a salt thereof

Process 5

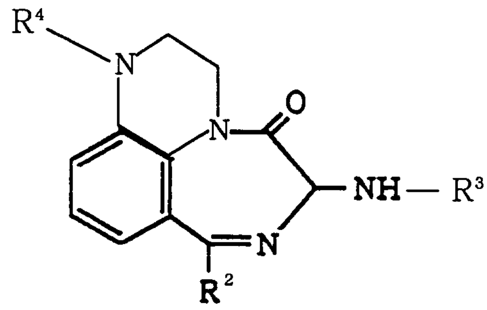

(IV)
or a salt thereof

Elimination of the imino protective group

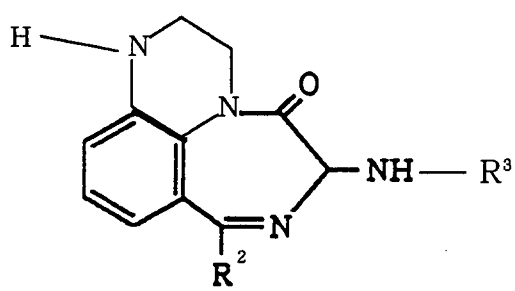

(Ig)
or a salt thereof

Process 6

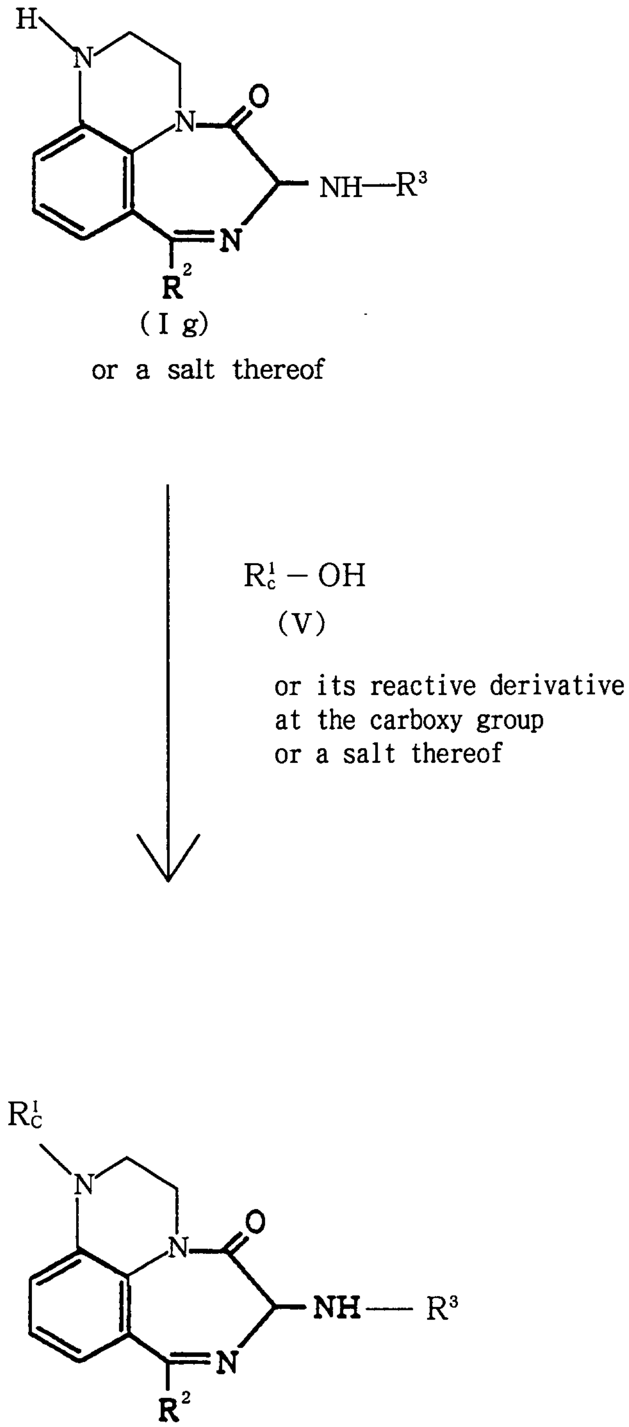


(I g)
or a salt thereof

(I h)
or a salt thereof

wherein

| | |
|---|---|
| $R^1$, $R^2$ and $R^3$ | are each as defined above, |
| $R_a^1$ | is protected carboxy,<br>protected carboxy(lower)alkyl,<br>protected carboxy(lower)alkanoyl,<br>protected carboxy(lower)alkenoyl, or |

heterocycliccarbonyl(lower)alkanoyl which has protected carboxy,

$R_b^1$ is carboxy, carboxy(lower)alkyl, carboxy(lower)alkanoyl, carboxy(lower)alkenoyl, or heterocycliccarbonyl(lower)alkanoyl which has carboxy,

A is lower alkylene,

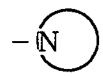

is piperazinyl or pyrrolidinyl, each of which may have suitable substituent(s)

$A^1$ is $(C_1\text{-}C_5)$ alkylene,

$R^4$ is imino-protective group,

$R_c^1$ is carboxy(lower)alkanoyl, protected carboxy(lower)alkanoyl, carboxy(lower)alkenoyl, protected carboxy(lower)alkenoyl, or heterocycliccarbonyl(lower)alkanoyl which may have suitable substituent(s).

The starting compound (II) is novel and can be prepared by the following process.

Process A

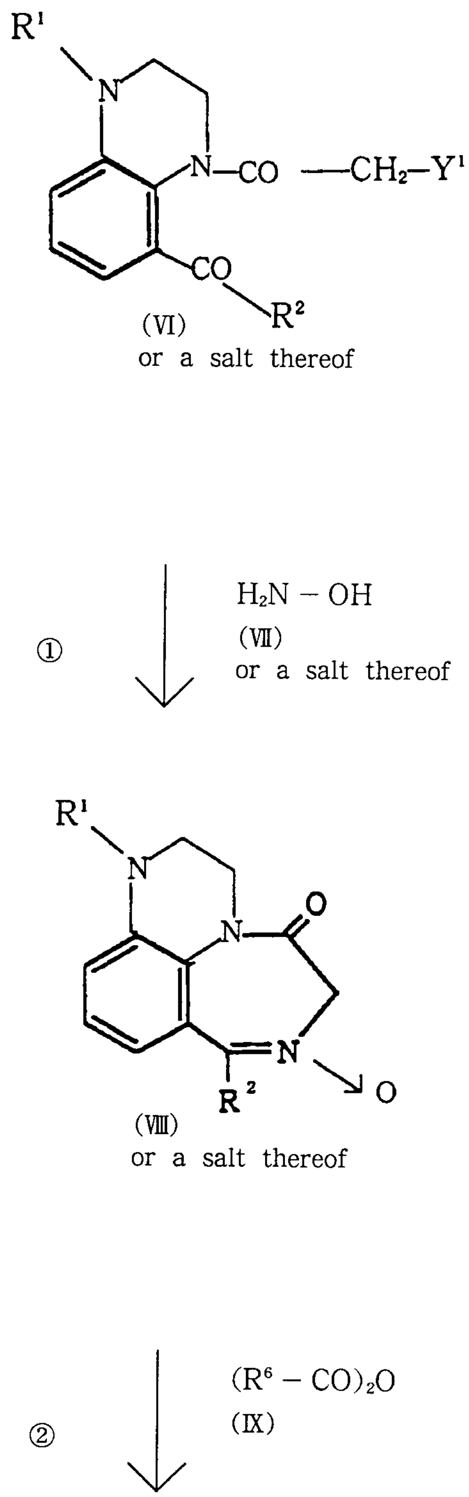


(VI)
or a salt thereof

(VIII)
or a salt thereof

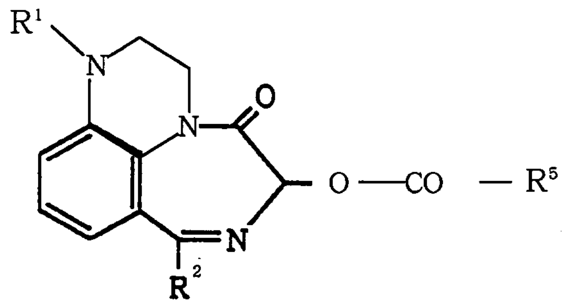

(X) or a salt thereof

③ Hydrolysis

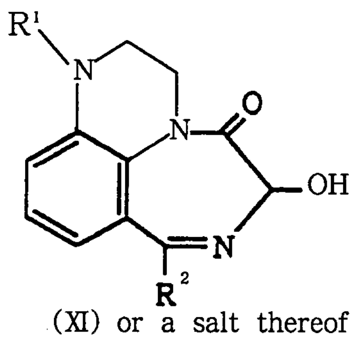

(XI) or a salt thereof

④ $R^6 - SO_2 - Y^2$
(XII)

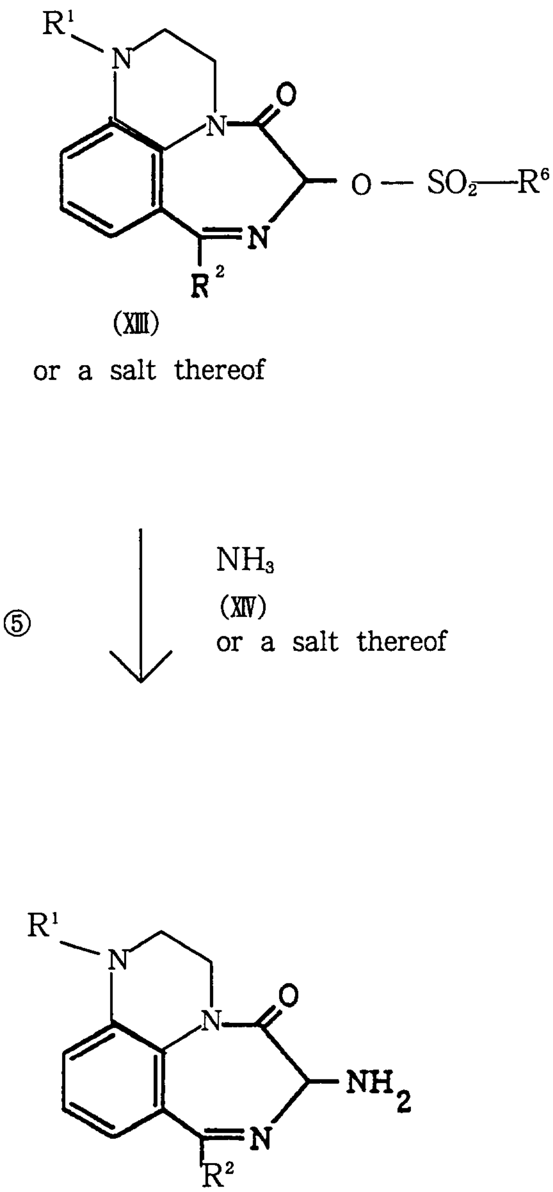


(XIII)

or a salt thereof

(II)

or a salt thereof

wherein

| | |
|---|---|
| $R^1$ and $R^2$ | are each as defined above, |
| $Y^1$ | is halogen, |
| $R^5$ | is lower alkyl, |
| $R^6$ | is lower alkyl, |
| $Y^2$ | is halogen. |

The starting compound (VI) or a salt thereof can be prepared by the procedures described in the Preparations mentioned later or by the similar manner.

Suitable salts of the compound (I) are conventional non-toxic, pharmaceutically acceptable salt and may includea salt with a base or an acid addition salt such as a salt with an inorganic base, for example, an alkaline metal salt [e. g. sodium salt, potassium salt, etc.], an alkaline earth metal salt [e. g. calcium salt, magnesium salt, etc.], an ammonium salt; a salt with an organic base, for example, an organic amine salt [ e. g. triethylamine salt, pyridine salt, picoline salt, ethanolamine salt, triethanolamine salt , dicyclohexylamine salt, N , N' - dibenzylethylenediamine salt, etc.]; an inorganic acid addition salt [e. g. hydrochloride,

hydrobromide, sulfate, phosphate, etc.]; an organic carboxylic or sulfonic acid addition salt [e. g. formate, acetate, trifluoroacetate, maleate, tartrate, methanesulfonate, benzenesulfonate, toluenesulfonate, etc.]; a salt with a basic or acidic amino acid [e. g. arginine salt, aspartic acid salt, glutamic acid salt, etc.];and the like.

In the above and subsequent descriptions of the present specification, suitable examples and illustrations of the various definitions to be included within the scope of the invention are explained in detail as follows.

The term "lower" is intended to mean 1 to 6 carbon atom(s), unless otherwise indicated.

The term "higher" is intended to mean 7 to 20 carbon atoms, unless otherwise indicated.

Suitable "protected carboxy" and "protected carboxy moiety" in the terms "protected carboxy(lower)-alkyl", "protected carboxy (lower)alkanoyl" and "protected carboxy(lower)alkenoyl" may include esterified carboxy, and the like.

Suitable "ester moiety" in "esterified carboxy" may include lower alkyl ester [e. g. methyl ester, ethyl ester, propyl ester, isopropyl ester, butyl ester, isobutyl ester, tert-butyl ester, pentyl ester, tert-pentyl ester, hexyl ester, 1-cyclopropylethyl ester, etc.]; lower alkenyl ester [e. g. vinyl ester, allyl ester, etc.]; lower alkynyl ester [e. g. ethynyl ester, propynyl ester, etc.]; lower alkoxy(lower)alkyl ester [e. g. methoxymethyl ester, ethoxymethyl ester, isopropoxymethyl ester, t-butoxymethyl ester, 1-methoxyethyl ester, 1-ethoxyethyl ester, etc.]; lower alkylthio(lower)alkyl ester [e. g. methylthiomethyl ester, ethylthiomethyl ester, ethylthioethyl ester, isopropylthiomethyl ester, etc.]; mono (or di or tri) halo (lower)alkyl ester [e. g. 2-iodoethyl ester, 2, 2, 2,- trichloroethyl ester, etc.]; lower alkanoyloxy(lower)alkyl ester [ e. g. acetoxymethyl ester, propionyloxymethy ester, butyryloxymethyl ester , valeryloxymethyl ester , pivaloyloxymethyl ester , hexanoyloxymethyl ester, 2- acetoxyethyl ester, 2-propionyloxyethyl ester, etc.]; lower alkanesulfonyl(lower) alkyl ester [e. g. mesylmethyl ester, 2-mesylethyl ester, etc.]; ar(lower)alkyl ester such as phenyl(lower)alkyl ester which may have one or more suitable substituent(s) [e. g. benzyl ester, 4-methoxybenzyl ester, 4-nitrobenzyl ester, phenetyl ester, trityl ester, benzhydryl ester, bis (methoxyphenyl)methyl ester, 3, 4-dimethoxybenzyl ester, 4-hydroxy-3, 5-di-t-butylbenzyl ester, etc.];aryl ester which may have one or more suitable substituent(s) such as phenyl ester which has substituent(s) and phenyl ester which has no substituent [e. g. phenyl ester, tolyl ester, t-butylphenyl ester, xylyl ester, mesityl ester, cumenyl ester, 4-chlorophenyl ester, 4-methoxyphenyl ester, etc.]; tri(lower)alkylsilyl ester; lower alkylthio ester [e. g. methylthio ester, ethylthio ester, etc.], and the like.

Suitable "lower alkyl" and "lower alkyl moiety" in the terms "carboxy(lower)alkyl", "protected carboxy-(lower)alkyl" and " heterocycliccarbonyl(lower) alkyl" may include straight or branched one, having 1 to 6 carbon atom(s), such as methyl, ethyl, propyl, isopropyl, butyl, isobutyl, t- butyl, pentyl, neopentyl, hexyl, in which the preferable one is $(C_1-C_4)$alkyl.

Suitable "lower alkanoyl moiety" in the terms "carboxy (lower)alkanoyl", "protected carboxy(lower)-alkanoyl" and " heterocycliccarbonyl(lower)alkanoyl" may include formyl, acetyl, propionyl, butanoyl, pentanoyl, hexanoyl, and the like.

Suitable "lower alkenoyl moiety" in the terms "carboxy (lower)alkenoyl" and "protected carboxy(lower)-alkenoyl" may include propenoyl, butenoyl, methacryloyl, pentenoyl, hexenoyl, and the like.

Suitable "aryl" of $R^2$ may include phenyl, naphthyl, and the like, and aforesaid aryl may have one or more (preferably 1 to 3) suitable substituent(s) such as halogen, amino, lower alkoxy and mono(or di or tri)-halo(lower)alkyl.

Suitable "halogen" and "halogen moiety" in "mono(or di or tri)halo(lower)alkyl" may include fluoro, chloro, bromo and iodo.

Suitable "lower alkoxy" may include straight or branched one such as methoxy, ethoxy, propoxy, isopropoxy, butoxy, t-butoxy, pentyloxy, and hexyloxy.

Suitable "acyl" may include carbamoyl, aliphatic acyl and acyl group containing an aromatic ring, which is reffered to as aromatic acyl, or an heterocyclic ring, which is reffered to as heterocyclic acyl.

This acyl group may be derived, for example, from an organic carboxylic acid, an organic carbonic acid, an organic sulfuric acid, an organic sulfonic acid and an organic carbamic acid.

Suitable example of said acyl may be illustrated as follows:

Carbamoyl;

Aliphatic acyl such as lower or higher alkanoyl [e. g. formyl, acetyl, propanoyl, butanoyl, 2-methylpropanoyl, pentanoyl, 2, 2-dimethylpropanoyl, hexanoyl, heptanoyl, octanoyl, nonanoyl, decanoyl, undecanoyl, dodecanoyl, tridacanoyl, tetradecanoyl, pentadecanoyl, hexadecanoyl, heptadecanoyl, octadecanoyl, nonadecanoyl, icosanoyl, etc.];

lower or higher cycloalkylcarbonyl [e. g. cyclopropylcarbonyl, cyclobutylcarbonyl, cyclopentylcarbonyl, cyclohexylcarbonyl, etc.];

lower or higher alkylsulfonyl [e. g. methylsulfonyl, ethylsulfonyl, etc.];

lower or higher alkoxysulfonyl [e. g. methoxysulfonyl, ethoxysulfonyl, etc.]; or the like;

Aromatic acyl such as aroyl [e. g. benzoyl, toluoyl, naphthoyl, etc.];

ar(lower)alkanoyl [e. g. phenyl(lower)alkanoyl (e. g. phenylacetyl, phenylpropanoyl, phenylbutanoyl, phenylisobutylyl, phenylpentanoyl, phenylhexanoyl, etc.), naphthyl(lower)alkanoyl (e. g. naphthylacetyl, naphthylpropanoyl, naphthylbutanoyl, etc.), etc.];

ar(lower)alkenoyl [e. g. phenyl(lower)alkenoyl (e. g. phenylpropenoyl, phenylbutenoyl, phenylmethacryloyl, phenylpentenoyl, phenylhexenoyl, etc.), naphthyl(lower)alkenoyl (e. g. naphylpropenoyl, naphthylbutenoyl, naphthylpentenoyl, etc.), etc.];

ar(lower)alkoxycarbonyl [e. g. phenyl(lower)alkoxycarbonyl (e. g. benzyloxycarbonyl, etc.), etc.];

aryloxycarbonyl [e. g. phenoxycarbonyl, naphthyloxycarbonyl, etc.];

aryloxy(lower)alkanoyl [e. g. phenoxyacetyl, phenoxypropionyl, etc.];

arylcarbamoyl [e. g. phenylcarbamoyl, etc.];

arylthiocarbamoyl [e. g. phenylthiocarbamoyl, etc.];

arylglyoxyloyl [e. g. phenylglyoxyloyl, naphthylglyoxyloyl, etc.];

arylsulfonyl [e. g. phenylsulfonyl, naphthylsulfonyl, etc.];

or the like;

Heterocyclic acyl such as heterocycliccarbonyl;

heterocyclic(lower)alkanoyl [e. g. thienylacetyl, thienylpropanoyl, thienylbutanoyl, thienylpentanoyl, thienylhexanoyl, thiazolylacetyl, thiadiazolylacetyl, tetrazolylacetyl, etc.];

heterocyclic(lower)alkenoyl [e. g. heterocyclicpropenoyl, heterocyclicbutenoyl, heterocyclicpentenoyl, heterocyclichexenoyl, etc.];

heterocyclicglyoxyloyl [e. g. thiazolylglyoxyloyl, thienylglyoxyloyl, etc.]; or the like.

"Heterocyclic moiety" in the terms "heterocycliccarbonyl", "heterocyclic(lower)alkanoyl", "heterocyclic-(lower)alkenoyl" and "heterocyclic glyoxyloyl" means saturated or unsaturated , monocyclic or polycyclic heterocyclic group containing at least one hetero-atom such as an oxygen, sulfur, nitrogen atom and the like.

Suitable "heterocyclic group" in the terms "heterocycliccarbonyl", "heterocyclic(lower)alkanoyl", "heterocyclic(lower)alkenoyl" and "heterocyclicglyoxyloyl" may include unsaturated 3 to 8-membered (more preferably 5 or 6-membered) heteromonocyclic group containing 1 to 4 nitrogen atom(s), for example, pyrrolyl, pyrrolinyl, imidazolyl, pyrazolyl, pyridyl and its N-oxide, dihydropyridyl, pyrimidyl, pyrazinyl, pyridazinyl, triazolyl [e. g. 4H-1, 2, 4-triazolyl, 1H-1, 2, 3-triazolyl, 2H-1, 2, 3-triazolyl, etc.], tetrazolyl [e. g. 1H-tetrazolyl, 2H-tetrazolyl, etc.], etc.;

saturated 3 to 8-membered (more preferably 5 or 6-membered) heteromonocyclic group containing 1 to 4 nitrogen atom(s), for example, pyrrolidinyl, imidazolidinyl, piperidino, piperazinyl, etc.;

unsaturated condensed heterocyclic group containing 1 to 4 nitrogen atom(s), for example, indolyl, isoindolyl, indolinyl, indolizinyl, benzimidazolyl, quinolyl, isoquinolyl, dihydroquinolyl, indazolyl, benzotriazolyl, etc.;

unsaturated 3 to 8-membered (more preferably 5 or 6-membered) heteromonocyclic group containing 1 to 2 oxygen atom(s) and 1 to 3 nitrogen atom(s), for example, oxazolyl, isoxazolyl, oxadiazolyl [e. g. 1, 2, 4-oxadiazolyl, 1, 3, 4-oxadiazolyl, 1, 2, 5-oxadiazolyl, etc.], etc.;

saturated 3 to 8-membered (more preferably 5 or 6-membered) heteromonocyclic group containing 1 to 2 oxygen atom(s) and 1 to 3 nitrogen atom(s), for example, morpholinyl, sydnonyl, etc.;

unsaturated condensed heterocyclic group containing 1 to 2 oxygen atom(s) and 1 to 3 nitrogen atom(s), for example, benzoxazolyl, benzoxadiazolyl, etc.;

unsaturated 3 to 8-membered (more preferably 5 or 6-membered) heteromonocyclic group containing 1 to 2 sulfur atom(s) and 1 to 3 nitrogen atom(s), for example, thiazolyl, isothiazolyl, thiadiazolyl [e. g. 1, 2, 3-thiadiazolyl, 1, 2, 4-thiadiazolyl,1, 3, 4-thiadiazolyl, 1, 2, 5-thiadiazolyl, etc.], dihydrothiazinyl, etc.;

saturated 3 to 8-membered (more preferably 5 or 6-membered) heteromonocyclic group containing 1 to 2 sulfur atom(s) and 1 to 3 nitrogen atom(s), for example, thiazolidinyl, etc.;

unsaturated 3 to 8-membered (more preferably 5 or 6-membered) heteromonocyclic group containing 1 to 2 sulfur atom(s), for example, thienyl, dihydrodithiinyl, dihydrodithionyl, etc.;

unsaturated condensed heterocyclic group containing 1 to 2 sulfur atom(s) and 1 to 3 nitrogen atom(s), for example, benzothiazolyl, benzothiadiazolyl, etc.;

unsaturated 3 to 8-membered (more preferably 5 or 6-membered) heteromonocyclic group containing an oxygen atom, for example, furyl, etc.;

unsaturated 3 to 8-membered (more preferably 5 or 6-membered) heteromonocyclic group containing an oxygen atom and 1 to 2 sulfur atom(s), for example, dihydrooxathiinyl, etc.;

unsaturated condensed heterocyclic group containing 1 to 2 sulfur atom(s), for example, benzothienyl [e. g.

benzo[b]thienyl, etc.], benzodithiinyl, etc.;
unsaturated condensed heterocyclic group containing an oxygen atom and 1 to 2 sulfur atom(s), for example, benzoxathiinyl, etc. and the like.

The acyl moiety as stated above may have 1 to 5, same or different, suitable substituent(s) such as halogen [e. g. fluorine, chlorine, bromine or iodine], lower alkyl [e. g. methyl, ethyl, propyl, isopropyl, butyl, isobutyl, t-butyl, pentyl, hexyl, etc.], lower alkoxy [e. g. methoxy, ethoxy, propoxy, isopropoxy, butoxy, t-butoxy, pentyloxy, hexyloxy, etc.], hydroxy, carboxy, protected hydroxy, protected carboxy, mono(or di or tri)halo(lower)alkyl, N, N-di(lower)alkylamino [e. g. N, N-dimethyamino, N, N-diethylamino, N, N-dipropylamino, N, N-dibutylamino, N, N-dipentylamino, N, N-dihexylamino, N-methyl-N-butylamino, etc.], or the like.

Suitable "substituent" in the terms "heterocyclic carbonyl(lower)alkyl which may have suitable substituent(s)" and "heterocycliccarbonyl(lower)alkanoyl which may have suitable substituent(s)" may include aforesaid halogen, carboxy, aforesaid lower alkyl, aforesaid protected carboxy, and the like.

Suitable "imino-protective group" may include acyl such as lower alkanoyl [e. g. formyl, acetyl, propionyl, pivaloyl, hexanoyl, etc.],
mono(or di or tri)halo(lower)alkanoyl [e. g. chloroacetyl, bromoacetyl, dichloroacetyl, trifluoroacetyl, etc.],
lower alkoxycarbonyl [e. g. methoxycarbonyl, ethoxycarbonyl, propoxycarbonyl, t-butoxycarbonyl, t-pentyloxycarbonyl, hexyloxycarbonyl, etc.],
lower alkenyloxycarbonyl [e. g. allyloxycarbonyl, etc.],
carbamoyl, aroyl [e. g. benzoyl, toluoyl, naphthoyl, etc.],
ar(lower)alkanoyl [e. g. phenylacetyl, phenylpropionyl, etc.],
aryloxycarbonyl [e. g. phenoxycarbonyl, naphthyloxycarbonyl, etc.],
aryloxy(lower)alkanoyl [e. g. phenoxyacetyl, phenoxypropionyl, etc.],
arylglyoxyloyl [e. g. phenylglyoxyloyl, naphthylglyoxyloyl, etc.] and
ar(lower)alkoxycarbonyl which may have suitable substituent(s) [e. g. benzyloxycarbonyl, phenethyloxycarbonyl, p-nitrobenzyloxycarbonyl, etc.];
ar(lower)alkylidene such as ar(lower)alkylidene which may have substituent(s) [e. g. benzylidene, hydroxybenzylidene, etc.];
mono(or di or tri)phenyl(lower)alkyl [e. g. benzyl, phenethyl, benzhydryl, trityl, etc.]; and the like,
in which more preferable example may be ($C_1$- $C_4$)alkoxycarbonyl, ($C_2$- $C_4$)alkenyloxycarbonyl and ar($C_1$-$C_4$)alkoxycarbonyl which may have nitro and the most preferable one may be methoxycarbonyl, allyloxycarbonyl and benzyloxycarbonyl.

Suitable "lower alkylene" may include straight one having 1 to 6 carbon atom(s), such as methylene, ethylene, trimethylene, tetramethylene, pentamethylene and hexamethylene.

Suitable "substituent" in the terms "piperazinyl or pyrrolidinyl, each of which may have suitable substituent(s)" may include aforesaid halogen, carboxy, aforesaid lower alkyl, aforesaid protected carboxy, and the like.

Suitable "heterocyclic group" in the terms "heterocyclic carbonyl(lower)alkyl" and "heterocyclic carbonyl(lower)alkanoyl" can be reffered to the ones as exemplified above.

The preferable compound of formula (I) will be described below.

Examples of $R^1$ are preferably hydrogen, carboxy, protected carboxy [more preferably esterified carboxy, most preferably lower alkoxycarbonyl, and mono- (or di- or tri-) phenyl (lower) alkoxycarbonyl], carboxy (lower) alkyl , protected carboxy (lower) alkyl [more preferably esterified carboxy (lower) alkyl, most preferably lower alkoxycarbonyl (lower) alkyl], carboxy (lower) alkanoyl, protected carboxy (lower) alkanoyl [ more preferably esterified carboxy (lower) alkanoyl, most preferably lower alkoxycarbonyl (lower) alkanoyl], carboxy (lower) alkenoyl , protected carboxy (lower) alkenoyl [more preferably esterified carboxy (lower) alkenoyl, most preferably lower alkoxycarbonyl (lower) alkenoyl], heterocycliccarbonyl (lower) alkyl which may have from one to three suitable substituents (more preferably one suitable substituent) [more preferably heterocycliccarbonyl (lower) alkyl which may have lower alkyl, most preferably pyrrolidinylcarbonyl ( lower ) alkyl, and lower alkyl piperazinylcarbonyl (lower) alkyl], and heterocycliccarbonyl (lower) alkanoyl which may have from one to three suitable substituents (more preferably one suitable substituent) [more preferably heterocycliccarbonyl (lower) alkanoyl which may have a substituent selected from the group consisting of lower alkyl, carboxy and protected carboxy ( more preferably esterified carboxy, most preferably mono- (or di- or tri-) phenyl (lower) alkoxycarbonyl ), most preferably lower alkyl piperadinylcarbonyl (lower) alkanoyl, carboxypyrrolidinylcarbonyl (lower) alkanoyl , or protected carboxypyrrolidinylcarbonyl (lower) alkanoyl].

Examples of $R^2$ are preferably aryl which may have from one to three suitable substituents (more preferably one suitable substituent) [more preferably phenyl which may have one suitable substituent, most

preferably phenyl and halophenyl].

Examples of $R^3$ are preferably heterocycliccarbonyl (more preferably indolylcarbonyl), and arylcarbamoyl which may have from one to three suitable substituents (more preferably one suitable substituent)-(more preferably phenylcarbamoyl which may have one suitable substituent, most preferably halophenylcarbamoyl).

The processes for preparing the object compound (I) of the present invention are explained in detail in the following.

Process 1

The object compound (I) or a salt thereof can be prepared by subjecting the compound (II) or its reactive derivatives at the amino group or a salt thereof to acylation reaction.

Suitable acylating agent to be used in the present acylation reaction may include the conventional one of the formula:

$R^3$-OH (XV)

(whetrein $R^3$ is acyl)
or its reactive derivative or a salt thereof.

Suitable reactive derivative at the amino group of the compound (II) may include Schiff's base type imino or its tautomeric enamine type isomer formed by the reaction of the compound (II) with a carbonyl compound such as aldehyde, ketone or the like; a silyl derivative formed by the reaction of the compound (II) with a silyl compound such as N, O- bis (trimethylsilyl)acetamide, N-(trimethyl-silyl)acetamide or the like; a derivative formed by reaction of the compound (II) with phosphorus trichloride or phosgene, and the like.

Suitable salts of the compound (II) and the compound (XV) can be reffered to the ones as exemplified for the compound (I).

Suitable reactive derivative of the compound (XV) may include an acid halide, an acid anhydride, an activated amide, an activated ester, isocyanate, and the like. The suitable example may be an acid chloride; an acid azide; a mixed acid anhydride with an acid such as substituted phosphoric acid [e. g. dialkylphosphoric acid, phenylphosphoric acid, diphenylphosphoric acid, dibenzylphosphoric acid, halogenated phosphoric acid, etc.], dialkylphosphorous acid, sulfurous acid, thiosulfuric acid, alkanesulfonic acid [e. g. methanesulfonic acid, ethanesulfonic acid, etc.], sulfuric acid, alkylcarbonic acid, aliphatic carboxylic acid [e. g. pivalic acid, pentanoic acid, isopentanoic acid, 2-ethylbutyric acid or trichloroacetic acid, etc.] or aromatic carboxylic acid [e. g. benzoic acid, etc.]; a symmetrical acid anhydride; a cyclic acid anhydride; an activated amide with imidazole, 4-substituted imidazole, dimethypyrazole, triazole or tetrazole; or an activated ester [e. g. cyanomethy ester, methoxymethyl ester, dimethyliminomethyl $[(CH_3)_2N^+=CH-]$ ester, vinyl ester, propargyl ester, p-nitrophenyl ester, 2, 4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethy thioester, pyranyl ester, pyridyl ester, piperidyl ester, 8-quinolyl thioester, etc.], or an ester with a N-hydroxy compound [e. g. N, N-dimethylhydroxylamine, 1-hydroxy-2-(1H)-pyridone, N-hydroxysuccinimide, N-hydroxyphthalimide, 1-hydroxy-6-chloro-1H-benzotriazole, etc.]; substituted or unsubstituted aryl isocyanate; substituted or unsubstituted lower alkyl isocyanate; and the like. These reactive derivatives can optionally be selected from them according to the kind of the compound (XV) to be used.

The reaction is usually carried out in a conventional solvent such as water, acetone, dioxane, acetonitrile, chloroform, methylene chloride, ethylene chloride, tetrahydrofuran, ethyl acetate, N, N-dimethylformamide, pyridine or any other organic solvent which does not adversely influence the reaction. These conventional solvents may also be used in a mixture with water.

When the compound (XV) is used in a free acid form or its salt form in the reaction, the reaction is preferably carried out in the presence of a conventional condensing agent such as N, N' -dicyclohexylcarbodiimide, N-cyclohexyl-N'-morpholinoethylcarbodiimide, N-cyclohexyl-N'-(4-diethylaminocyclohexyl)-carbodiimide, N, N'-diethylcarbodiimide, N, N'-diisopropylcarbodiimide, N-ethyl-N'-(3-dimethylaminopropyl)-carbodiimide, N, N'-carbonylbis-(2-methylimidazole), pentamethyleneketene-N-cyclohexylimine, diphenylketene-N-cyclohexylimine, ethoxyacetylene, 1-alkoxy-1-chloroethylene, trialkyl phosphite, ethyl polyphosphate, isopropyl polyphosphate, phosphorus oxychloride (phosphoryl chloride), phosphorus trichloride, thionyl chloride, oxalyl chloride, triphenyl phosphine, 2-ethyl-7-hydroxybenzisoxazolium salt, 2-ethyl-5-(m-sulfophenyl)isoxazolium hydroxide intramolecular salt, 1-(p-chlorobenzenesulfonyloxy)-6-chloro-1H-benzotriazole, so-called Vilsmeier reagent prepared by the reaction of N, N-dimethylformamide with thionyl

chloride, phosgene, phosphorus oxychloride, etc., or the like.

The reaction may also be carried out in the presence of an inorganic or organic base such as an alkali metal bicarbonate, tri(lower)alkylamine, pyridine, N-(lower)alkylmorpholine, N, N-di(lower)alkylbenzylamine, or the like.

The reaction temperature is not critical, and the reaction is usually carried out under cooling to heating.

Process 2

The compound (Ib) or a salt thereof can be prepared by subjecting the compound (Ia) or a salt thereof to elimination reaction of the carboxy protective group(s).

This reaction is carried out in accordance with a conventional method such as hydrolysis, reduction or the like.

(i)Hydrolysis

The hydrolysis is preferably carried out in the presence of a base or an acid.

Suitable base may include an inorganic base and an organic base such as an alkali metal [e. g. sodium, potassium, etc.], the hydroxide or carbonate or bicarbonate thereof, trialkylamine [e. g. trimethylamine, triethylamine, etc.], picoline, 1, 5-diazabicyclo[4. 3. 0]non-5-ene, 1, 4-diazabicyclo[2. 2. 2]octane, 1, 8-diazabicyclo[5. 4. 0]undec-7-ene, or the like.

Suitable acid may include an organic acid [e. g. formic acid, acetic acid, propionic acid, trichloroacetic acid, trifluoroacetic acid, etc.] and an inorganic acid [e. g. hydrochloric acid, hydrobromic acid, sulfuric acid, hydrogen chloride, hydrogen bromide, etc.]. The elimination using Lewis acid such as trihaloacetic acid [e. g. trichloroacetic acid, trifluoroacetic acid, etc.] or the like is preferably carried out in the presence of cation trapping agents [e. g. anisole, phenol, etc.].

The reaction is usually carried out in a solvent such as water, alcohol [e. g. methanol, ethanol, etc.], tetrahydrofuran, methylene chloride, a mixture thereof or any other solvent which does not adversely influence the reaction. A liquid base or acid can be also used as the solvent.

The reaction temperature is not critical, and the reaction is usually carried out under cooling to warming.

(ii)Reduction

The reduction is carried out in a conventional manner including chemical reduction and catalytic reduction.

Suitable reducing agents to be used in chemical reduction are a combination of a metal [e. g. tin, zinc, iron, etc.] or a metallic compound [e. g. chromium chloride, chromium acetate, etc.] and an organic or inorganic acid [e. g. formic acid, acetic acid, propionic acid, trifluoroacetic acid, p-toluenesulfonic acid, hydrochloric acid, hydrobromic acid, etc.].

Suitable catalysts to be used in catalytic reduction are conventional ones such as platinum catalysts [e. g. platinum plate, spongy platinum, platinum black, colloidal platinum, platinum oxide, platinum wire, etc.], palladium catalysts [e. g. spongy palladium, palladium black, palladium oxide, palladium on carbon, colloidal palladium, palladium on barium sulfate, palladium on barium carbonate, etc.], nickel catalysts [e. g. reduced nickel, nickel oxide, Raney nickel, etc.], cobalt catalysts [e. g. reduced cobalt, Raney cobalt, etc.], iron catalysts [e. g. reduced iron, Raney iron, etc.], copper catalysts [e. g. reduced copper, Raney copper, Ullman copper, etc.] and the like.

The reduction is usually carried out in a conventional solvent which does not adversely influence the reaction such as water, methanol, ethanol, propanol, N, N-dimethylformamide, tetrahydrofuran, or a mixture thereof. Additionally, in case that the above-mentioned acids to be used in chemical reduction are liquid, they can also be used as a solvent.

The reaction temperature of this reaction is not critical, and the reaction is usually carried out under cooling to warming.

Process 3

The object compound (Id) or a salt thereof can be prepared by reacting the compound (Ic) or its reactive derivative at the carboxy group or a salt thereof with the compound (III) or a salt thereof.

Suitable reactive derivative at the carboxy group of the compound (Ic) may include an acid halide, an acid anhydride, an activated amide, an activated ester, and the like. The suitable example of the reactive derivative may be an acid chloride; an acid aside; a mixed acid anhydride with an acid such as substituted phosphoric acid [e. g. dialkylphosphoric acid, phenylphosphoric acid, diphenylphosphoric acid, dibenzylphosphoric acid, halogenated phosphoric acid, etc.], dialkylphosphorous acid, lower alkanesulfonic acid [e. g. methanesulfonic acid, ethanesulfonic acid, etc.], sulfurous acid, thiosulfuric acid, sulfuric acid, aliphatic carboxylic acid [e. g. acetic acid, propionic acid, butyric acid, isobutyric acid, pivalic acid, valeric acid, isovaleric acid, 2 - ethylbutyric acid , trichloroacetic acid , etc . ] or aromaticcarboxylic acid [e. g. benzoic acid, etc.]; a symmetrical acid anhydride; an activated amide with imidazole, 4-substituted imidazole, dimethypyrazole, triazole or tetrazole; or an activated ester [e. g. cyanomethy ester, methoxymethyl ester, dimethyliminomethyl [$(CH_3)_2N^+=CH$-] ester, vinyl ester, propargyl ester, p-nitrophenyl ester, 2, 4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethy thioester, pyranyl ester, pyridyl ester, piperidyl ester, 8-quinolyl thioester, etc.], or an ester with a N-hydroxy compound [e. g. N, N-dimethylhydroxylamine, 1-hydroxy-2-(1H)-pyridone, N-hydroxysuccinimide, N-hydroxyphthalimide, 1-hydroxy-1H-benzotriazole, etc.], and the like. These reactive derivatives can optionally be selected from them according to the kind of the compound (Ic) to be used.

The reaction is usually carried out in a conventional solvent such as water, alcohol [e. g. methanol, ethanol, etc.], acetone, dioxane, acetonitrile, chloroform, methylene chloride, ethylene chloride, tetrahydrofuran, ethyl acetate, N, N-dimethylformamide, pyridine or any other organic solvent which does not adversely influence the reaction. These conventional solvents may also be used in a mixture with water.

When the compound (Ic) is used in a free acid form or its salt form in the reaction, the reaction is preferably carried out in the presence of a conventional condensing agent such as N, N'-dicyclohexylcarbodiimide, N-cyclohexyl-N'-morpholinoethylcarbodiimide, N-cyclohexyl-N'-(4-diethylaminocyclohexyl)-carbodiimide, N, N'-diethylcarbodiimide, N, N'-diisopropylcarbodiimide, N-ethyl-N'-(3-dimethylaminopropyl)-carbodiimide, N, N'-carbonylbis-(2-methylimidazole), pentamethyleneketene-N-cyclohexylimine, diphenylketene-N-cyclohexylimine, ethoxyacetylene, 1-alkoxy-1-chloroethylene, trialkyl phosphite, ethyl polyphosphate, isopropyl polyphosphate, phosphorus oxychloride (phosphoryl chloride), phosphorus trichloride, thionyl chloride, oxalyl chloride, lower alkyl haloformate [e. g. ethyl chloroformate, isopropyl chloroformate, etc.], triphenyl phosphine, 2-ethyl-7-hydroxybenzisoxazolium salt, 2-ethyl-5-(m-sulfophenyl)isoxazolium hydroxide intramolecular salt, 1-(p-chlorobenzenesulfonyloxy)-6-chloro-1H-benzotriazole, so-called Vilsmeier reagent prepared by the reaction of N, N-dimethylformamide with thionyl chloride, phosgene, phosphorus oxychloride, etc., or the like.

The reaction may also be carried out in the presence of an inorganic or organic base such as an alkali metal bicarbonate, tri(lower)alkylamine, pyridine, N-(lower)alkylmorpholine, N, N-di(lower)alkylbenzylamine, or the like.

The reaction temperature is not critical, and the reaction is usually carried out under cooling to warming.

Process 4

The object compound (If) or a salt thereof can be prepared by reacting the compound (Ie) or its reactive derivative at the carboxy group or a salt thereof with the compound (III) or a salt thereof.

The reaction mode and reaction conditions of this reaction are to be reffered to those as explained in Process 3.

Process 5

The compound (Ig) or a salt thereof can be prepared by subjecting the compound (IV) or a salt thereof to elimination reaction of the imino protective group(s).

The reaction mode and reaction conditions of this reaction are to be reffered to those as explained in Process 2.

Process 6

The object compound (Ih) or a salt thereof can be prepared by reacting the compound (Ig) or a salt thereof with the compound (V) or its reactive derivative at the carboxy group or a salt thereof.

The reaction mode and reaction conditions of this reaction are to be reffered to those as explained in Process 3.

The processes for preparing the starting compound (II) are explained in the following.

Process A-①

The compound (VIII) or a salt thereof can be prepared by reacting the compound (VI) or a salt thereof with the compound (VII) or a salt thereof.

This reaction can be carried out according to the methods disclosed later in Preparations of the present specification or the similar manners thereto.

Process A-②

The compound (X) or a salt thereof can be prepared by reacting the compound (VIII) or a salt thereof with the compound (IX).

The reaction can be carried in the presence of a conventional solvent or without solvent.

The reaction temperature is not critical, and the reaction is usually carried out at ambient temperature or under warming or heating.

Process A-③

The compound (XI) or a salt thereof can be prepared by subjecting the compound (X) or a salt thereof to hydrolysis .

The hydrolysis can be carried out in the presence of a base. Suitable base may include an inorganic base such as an alkali metal hydroxide [e. g. sodium hydroxide, potassium hydroxide, etc.], an alkaline earth metal hydroxide [e. g. magnesium hydroxide, calcium hydroxide, etc.], an alkali metal carbonate [e. g. sodium carbonate, potassium carbonate, etc.], an alkaline earth metal carbonate [e. g. magnesium carbonate, calcium carbonate, etc.], and the like.

The reaction is usually carried out in a solvent such as water, alcohol [e. g. methanol, ethanol, etc.], benzene, N, N-dimethylformamide, tetrahydrofuran, diethyl ether or any other solvent which does not adversely influence the reaction.

The reaction temperature is not critical, and the reaction is usually carried out under cooling to warming.

Process A-④

The compound (XIII) or a salt thereof can be prepared by reacting the compound (XI) or a salt thereof with the compound (XII).

The reaction is usually carried out in the presence of a base such as tri(lower)alkylamine [e. g. trimethylamine, triethylamine, diisopropylamine etc.], di(lower)alkylaniline [e. g. dimethylaniline, etc.], and the like.

The reaction is usually carried out in a solvent such as benzene, N, N-dimethylformamide, tetrahydrofuran, methylene chloride, ethylene chloride, chloroform, diethyl ether or any other solvent which does not adversely influence the reaction.

The reaction temperature is not critical, and the reaction is usually carried out under cooling to warming.

Process A-⑤

The compound (II) or a salt thereof can be prepared by reacting the compound (XIII) or a salt thereof with the compound (XIV) or a salt thereof.

The reaction is usually carried out in a solvent such as alcohol [e. g. methanol, ethanol, etc.], benzene, N, N-dimethylformamide, tetrahydrofuran, methylene chloride, ethylene chloride, chloroform, diethyl ether or any other solvent which does not adversely influence the reaction.

The reaction temperature is not critical, and the reaction is usually carried out under cooling to warming.

The object compound (I) or the pharmaceutically acceptable salt thereof is a CCK antagonist and therefore useful as an agent for preventing and/or treating vomiting, pancreatitis and the like as mentioned

above.

In order to prove the usefulness of the object compound (I), pharmaceutical activities of the typical compounds thereof are mentioned below.

Test compound:

1-[3-[(2S)-2-carboxypyrrolidin-1-yl)carbonyl]propionyl]-5-oxo-6-(2-indolylcarbonylamino)-8-phenyl-2, 3, 5, 6-tetrahydro-1H-[1, 4]diazepino[1, 7, 6-de]quinoxaline

Test results:

Activity of the test compound that inhibits binding of [125I] CCK-8 to a rat pancreatic CCK receptor

Table 1

| Test compound | Inhibition (%) |
|---|---|
| $10^{-8}$ M | 96.7 |

The object compound (I) or the pharmaceutically acceptable salt thereof in the present invention can be administered to mammals including human being in the form of an ordinary preparation such as a capsule, microcapsule, tablets, granules, powder, troche, syrup, aerosol, inhalant, solution, injection, suspension, emulsion or suppository.

The pharmaceutical composition of the present invention can contain a vehicle such as a cane sugar, starch, mannitol, sorbitol, lactose, glucose, cellulose, talc, calcium phosphate or calcium carbonate; a binder such as cellulose, methyl cellulose, hydroxypropyl cellulose, polypropylpyrrolidone, gelatin, acacia, polyethylene glycol, cane sugar or starch; a disintegrant such as starch, carboxymethyl cellulose, calcium salt of carboxymethyl cellulose, hydroxypropyl cellulose, glycol starch sodium, sodium bicarbonate, calcium phosphate or calcium citrate; a lubricant such as magnesium stearate, talc or sodium lauryl phosphate; a flavor such as citric acid, menthol, glycerol or orange powder; a preservative such as sodium benzoate, sodium hydrogensulfite, methylparaben or propylparaben; a stabilizer such as citric acid, sodium citrate or acetic acid; a suspension such as methyl cellulose, polyvinyl pyrrolidone or aluminum stearate ; a dispersant; an aqueous diluent such as water; and an organic or inorganic carrier which is ordinarily used for medicines, such as cocoa butter, polyethylene glycol or illuminating kerosine.

The active ingredient may be administered from one to four times a day at a unit dose of from 0.01 to 50 mg/kg. The dose may vary depending on the age, weight and conditions of patients and on the administration method.

The following Preparations and Examples are given for the purpose of illustrating the present invention in more detail.

Preparation 1

To a solution of 1, 2, 3, 4- tetrahydroquinoxaline(4.02g) and 2-fluorobenzonitrile (4.36g) in dry toluene (30ml) was added dropwise a 16% solution of borontrichloride in toluene (24.2g) under stirring and cooling in an ice-bath. The mixture was stirred for 1 hour at ambient temperature. To the reaction mixture was added aluminium chloride (4.40g) gradually under ice-bath cooling. After being stirred for 0.5 hour at ambient temperature, the mixture was refluxed for 4.5 hours. To the reaction mixture were added water (7ml) and 2N HCl (22ml) and the resultant mixture was refluxed for 2.5 hours. Water and ethyl acetate were added to the reaction mixture and the separated organic layer was washed twice with water, dried over magnesium sulfate and then evaporated in vacuo. The residue was subjected to column chromatography on silica gel eluting with chloroform to give pure 5-(2-fluorobenzoyl)-1, 2, 3, 4-tetrahydroquinoxaline (4.14g) as a red oil.

IR(Film):3310, 1610, 1512, 1300, 1274, 1214, 750cm$^{-1}$

NMR($CDCl_3$)$\delta$:3.40(2H, t, J=4.4Hz), 3.64(2H, t, J=4.4Hz), 6.33 (1H, t, J=7.8Hz), 6.57(1H, d, J=7.8Hz), 6.74(1H, d, J=7.8Hz), 7.0 -7.5(5H, m), 8.86(1H, br, s)

Preparation 2

5-Benzoyl-1, 2, 3, 4-tetrahydroquinoxaline was obtained according to a similar manner to that of preparation 1.
NMR($CDCl_3$)δ:3.25-3.45(2H, m), 3.56-3.65(2H, m), 3.87(1H, s), 6.36(1H, d, J=7.6Hz), 6.59(1H, d, J=6.9Hz), 6.89(1H, dd, J=1.4Hz, 8.2Hz), 7.35-7.65(5H, m), 8.53(1H, s)

Preparation 3

To a solution of 5-(2-fluorobenzoyl)-1, 2, 3, 4-tetrahydroquinoxaline (6.40g) in methyl isobutyl ketone (120ml) were added potassium carbonate (3.45g) and ethy bromoacetate (6.26g) successively under stirring. The mixture was refluxed for 5.5 hours with stirring and allowed to stand at ambient temperature overnight. An additional ethy bromoacetate (1.05g) was added to the mixture and the mixture was refluxed for 3.5 hours further. Ethyl acetate and water were added to the reaction mixture with vigorous stirring. The separated organic layer was washed twice with water, dried over magnesium sulfate and then evaporated in vacuo. The residue was crystallized on standing and was washed with diisopropyl ether. Collection by filtration gave pure 1-ethoxycarbonylmethyl-5-(2-fluorobenzoyl)-1, 2, 3, 4-tetrahydroquinoxaline (6.19g) as an orange crystal. The filtrate was subjected to column chromatography on silica gel eluting with chloroform to give an additional crop of the objective compound (2.20g).
Mass(m/e):342($M^+$)
mp: 147-148 °C
IR(Nujol):3280, 1736, 1608cm$^{-1}$
NMR($CDCl_3$)δ:1.27(3H, t, J=7.0Hz), 3.49(2H, t, J=4.4Hz), 3.69 (2H, t, J=4.4Hz), 4.01(2H, s), 4.21(2H, q, J=7.0Hz), 6.3-6.5(2H, m), 6.7-6.8(1H, m), 7.0-7.45(5H, m), 9.25(1H, br. s)

Preparation 4

A solution of 5-benzoyl-1, 2, 3, 4-tetrahydroquinoxaline (29.5g) and di-tert-butyl carbonate (34g) in chloroform was refluxed for 30 hours. The reaction mixture was evaporated in vacuo. The residue was recrystallized from n-hexane to give 5-benzoyl-1-tert-butoxycarbonyl-1, 2, 3, 4-tetrahydroquinoxaline (38.2g).
mp: 115-117 °C
IR(Nujol):3320, 1690, 1620, 1580, 1505cm$^{-1}$
NMR($CDCl_3$)δ:1.53(9H, s), 3.54-3.60(2H, m), 3.76-3.82(2H, m), 6.50(1H, t, J=8.0Hz), 7.23-7.28(1H, m), 7.39-7.62(6H, m), 8.86 (1H, s)

Preparation 5

To a solution of 1-ethoxycarbonylmethyl-5-(2-fluorobenzoyl)-1, 2, 3, 4-tetrahydroquinoxaline (340.1mg) and pyridine (120mg) in dichloromethane (10ml) was added dropwise a solution of bromoacetyl bromide (240.8mg) in dichloromethane (1ml) under stirring and cooling in an ice-bath. The mixture was stirred under the same conditions for 1 hour and the additional bromoacetyl bromide (240.8mg) and pyridine (200mg) were added to the reaction mixture. The resultant mixture was stirred at ambient temperature for 1.5 hours further. To the reaction mixture was added chloroform and washed twice with water. The separated organic layer was dried over magnesium sulfate and evaporated in vacuo. The residue was subjected to column chromatography on silica gel eluting with chloroform to give 1-ethoxycarbonylmethyl-5-(2-fluorobenzoyl)-4-bromoacetyl-1, 2, 3, 4-tetrahydroquinoxaline (440mg) as a red oil. Triturating in diisopropyl ether containing a small amount of methanol gave an orange crystal, which was collected by filtration, washed with diisopropyl ether and dried to give pure product as an orange powder (267.5mg).
Mass(m/e):462($M^+$ -1), 463($M^+$), 463($M^+$ +1), 464($M^+$ +2)
mp: 117-118 °C
IR(Nujol):1740, 1656, 1608cm$^{-1}$
NMR($CDCl_3$)δ:1.28(3H, t, J=7.2Hz), 2.85(1H, dt, J=4.4Hz, 12.4Hz), 3.3-4.0(2H, m), 3.71(2H, s), 3.99(2H, s), 4.23(2H, q, J=7.2Hz), 4.58(1H, dd, J=4.4Hz, 12.8Hz), 6.7-7.85(7H, m)

Preparation 6

5-Benzoyl-4-bromoacetyl-1-tert-butoxycarbonyl-1, 2, 3, 4 -tetrahydroquinoxaline was obtained according to a similar manner to that of preparation 5.

mp: 63-75 °C
IR(Nujol):1700, 1660, 1590, 1580cm$^{-1}$
NMR($CDCl_3$)δ:1.53, 1.56(9H, s), 2.78-2.93(1/2H, m), 3.63-4.06 (5H, m), 4.60-4.70(1/2H, m), 7.05-8.11(8H, m)

Preparation 7

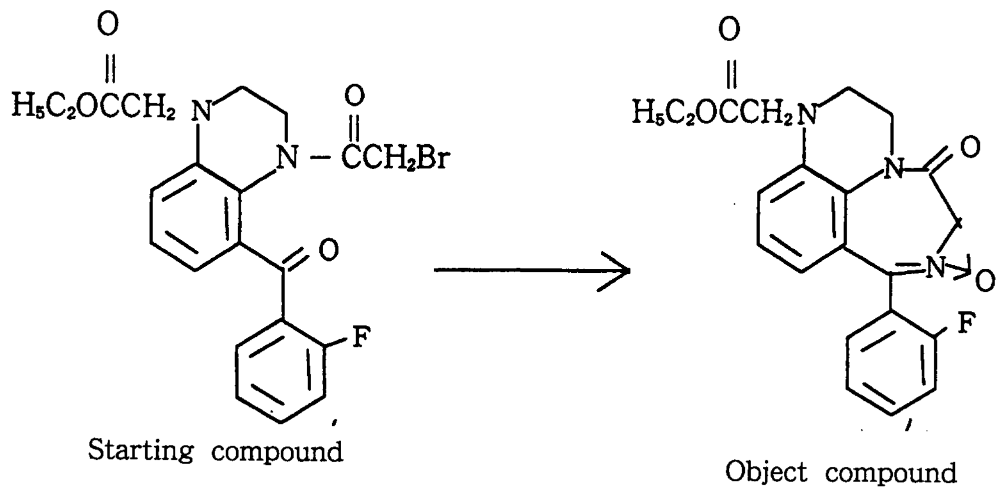

Starting compound

Object compound

A mixture of 1-ethoxycarbonylmethyl-5-(2-fluorobenzoyl)-4 - bromoacetyl- 1, 2, 3, 4- tetrahydroquinoxaline (231.5mg), hydroxylamine hydrochloride (53.7mg) and triethylamine (151.8mg) in ethanol (5ml) was warmed at 70 °C under stirring for 1.5 hours. Removal of the solvent in vacuo gave a residue, to which were added water and ethyl acetate under vigorous stirring. The separated organic layer was washed twice with water, dried over magnesium sulfate and evaporated in vacuo. The residue was subjected to column chromatography on silica gel eluting with chloroform to give an amorphous mass. Triturating in a mixture of methanol and diisopropyl ether gave a yellow crystal, which was collected by filtration and washed with diisopropyl ether to afford 1-ethoxycarbonylmethyl-5-oxo-8-(2-fluorophenyl)-2, 3, 5, 6-tetrahydro-1H-[1, 4]-diazepino[1, 7, 6-de]quinoxaline-7-oxide (93.2mg) as a yellow powder.
Mass(m/e):397($M^+$), 381($M^+$ -16)
mp: 197-198 °C
IR(Nujol):1742, 1685, 752cm$^{-1}$
NMR($CDCl_3$)δ:1.29(3H, t, J=7.2Hz), 3.13(1H, dt, J=3.6Hz, 12Hz), 3.57(1H, dd, J=2.0Hz, 10.4Hz), 3.72(1H, dt, J=4.0Hz, 12Hz), 4.11 (2H, dd, J=18.2Hz, 41.8Hz), 4.23(2H, q, J=7.2Hz), 4.77(2H, dd, J=18.2Hz, 41.8Hz), 4.99(1H, dd, J=2.4Hz, 12.8Hz), 6.39(1H, d, J=8Hz), 6.85(1H, d, J=8Hz), 6.99(1H, t, J=8Hz), 7.08-7.5(4H, m)

Preparation 8

A solution of 5 - benzoyl - 4 - bromoacetyl - 1 - tert - butoxycarbonyl-1, 2, 3, 4-tetrahydroquinoxaline (56g) in ethanol (300ml) was added dropwise to a mixture of hydroxylamine hydrochloride (43.6g) and sodium hydroxide (24g) in a mixture of water (200ml) and ethanol (218ml) under warming at 50~55 °C for a period of 5 minutes. The mixture was heated at 55~60 °C for 45 minutes with stirring and then treated with 12N hydrogen chloride (168ml) for 5 minutes at 55~65 °C and heated at 60~65 °C for 10 minutes. After removal of ethanol, the mixture was extracted with chloroform and then the separated organic layer was washed with water and a saturated aqueous solution of sodium bicarbonate, dried over magnesium sulfate and evaporated in vacuo. The residue was suspended in hot ethanol and the resultant precipitate was collected by filtration to give 5 -oxo-8-phenyl-2, 3, 5, 6-tetrahydro-1H-[1, 4]diazepino[1, 7, 6-de] quinoxaline-7-oxide (15.1g).
mp: 250-251 °C
IR(Nujol):3290, 1670, 1590, 1540, 1520cm$^{-1}$
NMR(DMSO-$d_6$)δ:2.84-2.97(1H, m), 3.23-3.31(1H, m), 3.51-3.57(1H, m), 4.35(1H, d, J=12.7Hz), 4.68-4.74-

(1H, m), 4.91(1H, d, J = 12.7Hz), 6.12(1H, dd, J = 1.3Hz, 7.7Hz), 6.64(1H, s), 6.72(1H, dd, J = 1.3Hz, 7.7Hz), 6.93(1H, t, J = 7.7Hz), 7.30-7.44(3H, m), 7.56-7.62(2H, m)

Preparation 9

To a solution of 5-oxo-8-phenyl-2, 3, 5, 6-tetrahydro-1H -[1, 4]diazepino[1, 7, 6-de]quinoxaline-7-oxide (0.20g) and pyridine (0.083ml) in dry dichloromethane (8ml) was added dropwise carbobenzoxy chloride (0.12ml) under stirring and cooling at 0 °C. After being stirred for 2 hours under the same conditions, the mixture was washed with water and a saturated aqueous solution of sodium bicarbonate. The separated organic layer was dried over magnesium sulfate and evaporated in vacuo. The residue was crystallized by triturating in diethyl ether to afford 1-benzyloxycarbonyl-5-oxo-8-phenyl-2, 3, 5, 6-tetrahydro-1H-[1, 4] diazepino[1, 7, 6-de]quinoxaline-7-oxide (0.19g).
IR(Nujol):1700, 1690, 1600, 1580, 1550, 1530cm$^{-1}$
NMR($CDCl_3$)δ:3.39-3.52(1H, m), 3.73-3.86(1H, m), 4.25-4.38 (1H, m), 4.60-4.74(1H, m), 4.72(2H, ABq, J = 12.4Hz), 5.28(2H, ABq, J = 9.97Hz), 6.90(1H, dd, J = 1.39Hz, 8.04Hz), 7.16(1H, t, J = 8.04Hz), 7.30-7.45-(8H, m), 7.65-7.72(2H, m), 7.96(1H, d, J = 8.04Hz)

Preparation 10

1-methoxycarbonyl-5-oxo-8-phenyl-2, 3, 5, 6-tetrahydro-1H -[1, 4]diazepino[1, 7, 6-de]quinoxaline-7-oxide was obtained according to a similar manner to that of preparation 9.
mp: 110-108 °C
IR(Nujol):1710, 1680, 1590, 1530cm$^{-1}$
NMR($CDCl_3$)δ:3.40-3.55(2H, m), 3.70-3.90(1.5H, m), 3.86(3H, s), 4.21-4.37(1H, m), 4.63-4.81(2.5H, m), 6.90-(1H, dd, J = 1.3Hz, 8.0Hz), 7.21(1H, t, J = 8.0Hz), 7.30-7.41(4H, m), 7.65-7.71(1H, m), 7.94(1H, d, J = 7.1Hz)

Preparation 11

A mixture of 1-ethoxycarbonylmethyl-5-oxo-8-(2-fluorophenyl)-2, 3, 5, 6-tetrahydro-1H-[1, 4]diazepino[1, 7, 6-de]quinoxaline-7-oxide (1.60g) and acetic anhydride (10ml) was heated at 100 °C under stirring for 10 minutes. The reaction mixture was poured in water and neutralized with sodium bicarbonate. The mixture was extracted twice with ethyl acetate and the combined extract was washed twice with water, dried over magnesium sulfate and evaporated in vacuo. The residue was subjected to column chromatography on silica gel eluting with chloroform to give 1-ethoxycarbonylmethyl-5-oxo-6-acetoxy-8-(2-fluorophenyl)-2, 3, 5, 6-tetrahydro-1H-[1, 4]diazepino [1, 7, 6-de]quinoxaline (1.95g) as a yellow powder, which was triturated in diisopropyl ether and collected by filtration to give pure product (1.29g).
Mass(m/e):439($M^+$), 366($M^+$ -73)
mp: 161-162 °C
IR(Nujol):1740, 1730, 1692, 1610, 1578cm$^{-1}$
NMR($CDCl_3$)δ:1.27(3H, t, J = 7.2Hz), 2.32(3H, s), 3.10(1H, dt, J = 5.2Hz, 12Hz), 3.54(1H, dd, J = 2.0Hz, 8.2Hz), 3.67(1H, dt, J = 5.2Hz, 8.2Hz), 4.10(2H, dd, J = 18Hz, 21.8Hz), 4.23(2H, q, J = 7.2Hz), 4.96 (1H, dd, J = 2.0Hz, 12Hz), 6.06(1H, s), 6.56(1H, d, J = 7.6Hz), 6.68(1H, d, J = 7.6Hz), 6.9-7.7(5H, m)

Preparation 12

To a suspended solution of 1-ethoxycarbonylmethyl-5-oxo-6-acetoxy-8-(2-fluorophenyl)-2, 3, 5, 6-tetrahydro-1H-[1, 4] diazepino [1, 7, 6-de]quinoxaline (219.7mg) in ethanol (11ml) was added dropwise 0.1N aqueous solution of sodium hydroxide (5ml) under stirring and cooling below 5 °C.

The suspension was stirred under the same conditions for 1.5 hours. The reaction mixture was neutralized with dil. HCl under cooling and adjusted to pH 6.0 with an aqueous solution of sodium bicarbonate. Ethanol was removed in vacuo from the reaction mixture and the residue was extracted twice with ethyl acetate. The combined extract was washed with water, dried over magnesium sulfate and evaporated in vacuo. The residue was crystallized on standing, triturated in diisopropyl ether and collected by filtration to give 1-ethoxycarbonylmethyl-5-oxo-6-hydroxy-8-(2-fluorophenyl)-2, 3, 5, 6-tetrahydro-1H-[1, 4] diazepino [1, 7, 6-de]quinoxaline (87.7mg)(1) as a yellow powder.

The aqueous layer was made acidic with dil. aqueous HCl and extracted twice with ethyl acetate. The combined extract was washed with water, dried over magnesium sulfate and evaporated in vacuo. The residue was crystallized by trituration in a mixture of methanol and diisopropyl ether and collected by

filtration to give pure 1-carboxymethyl-5-oxo-6-hydroxy-8-(2-fluorophenyl)-2, 3, 5, 6-tetrahydro-1H-[1, 4]-diazepino [1, 7, 6 -de]quinoxaline (77.0mg)(2) as a yellow powder.

(1)
Mass(m/e):397($M^+$), 368($M^+$ -29)
mp: 157-162 °C
IR(Nujol):3400, 1731, 1668, 1605$cm^{-1}$
NMR($CDCl_3$)$\delta$:1.27(3H, t, J=7.2Hz), 3.18(1H, dt, J=3.6Hz, 12Hz), 3.55(1H, dd, J=1.8Hz, 34Hz), 3.74(1H, dt, J=4.0Hz, 11.4Hz), 4.07 (2H, dd, J=18.2Hz, 48Hz), 4.20(2H, q, J=7.2Hz), 4.74(1H, br. s), 4.96(1H, dd, J=1.8Hz, 11.4Hz), 5.10(1H, s), 6.57(1H, d, J=7.6Hz), 6.64(1H, d, J=7.6Hz), 7.01(1H, t, J=7.6Hz), 7.2-7.75(4H, m)

(2)
Mass(m/e):369($M^+$), 340($M^+$ -29)
mp: 228 °C(dec.)
IR(Nujol):3400, 1710(br.), 1676, 1655, 1610$cm^{-1}$
NMR(DMSO-$d_6$)$\delta$:2.95(1H, br. t, J=8.2Hz), 3.34-3.52(3H, m), 4.20(2H, dd, J=18.4Hz, 45.4Hz), 4.82(2H, m), 6.36-7.58(7H, m), 12.82(1H, br. s)

Preparation 13

To a suspended solution of 1-ethoxycarbonylmethyl-5-oxo-6-acetoxy-8-(2-fluorophenyl)-2, 3, 5, 6-tetrahydro-1H-[1, 4] diazepino [1, 7, 6-de]quinoxaline (1.0417g) in ethanol (25ml) was added 0.1N aqueous solution of sodium hydroxide (30ml) under stirring and cooling in an ice-bath. The resultant clear solution was stirred for 2.5 hours at ambient temperature and for additional 1 hour at 50 °C.

Ethanol was removed in vacuo to give an aqueous solution, which was washed twice with ethyl acetate and then adjusted to pH 3 with a 6N aqueous solution of HCl. The aqueous mixture was extracted twice with ethyl acetate and the combined extract was washed with water, dried over magnesium sulfate and evaporated in vacuo. The residue was triturated in diisopropyl ether and collected by filtration to give pure 1-carboxymethyl-5-oxo-6-hydroxy-8-(2-fluorophenyl)-2, 3, 5, 6-tetrahydro-1H-[1, 4]diazepino [1, 7, 6-de]-quinoxaline (0.66g).

Mass(m/e):369($M^+$), 340($M^+$ -29)
mp: 228 °C(dec.)
IR(Nujol):3400, 1710( br.), 1676, 1655, 1610$cm^{-1}$
NMR(DMSO-$d_6$)$\delta$:2.95(1H, br. t, J=8.2Hz), 3.34-3.52(3H, m), 4.20(2H, dd, J=18.4Hz, 45.4Hz), 4.82(2H, m), 6.36-7.58(7H, m), 12.82(1H, br. s)

Preparation 14

A mixture of 1-benzyloxycarbonyl-5-oxo-8-phenyl-2, 3, 5, 6-tetrahydro-1H-[1, 4]diazepino[1, 7, 6-de]-quinoxaline-7-oxide (0.17g) and trifluoroacetic anhydride (0.17ml) was stirred at ambient temperature for 2 hours. The mixture was evaporated in vacuo and the residue was dissolved in a suspended mixture of dichloromethane and a saturated aqueous solution of sodium bicarbonate. The separated organic layer was dried over magnesium sulfate and evaporated in vacuo. The residue was crystallized from methanol to give 1-benzyloxycarbonyl-5-oxo-6-hydroxy-8-phenyl-2, 3, 5, 6-tetrahydro-1H-[1, 4]diazepino[1, 7, 6-de] quinoxaline (95mg).

mp: 175-178 °C
IR(Nujol):3600, 1710, 1690, 1600, 1670$cm^{-1}$
NMR($CDCl_3$)$\delta$:3.43-3.56(1H, m), 3.65-3.82(1H, m), 4.32(1H, quIntet, J=6.5Hz), 4.61(1H, quIntet, J=6.5Hz), 5.05(1H, s), 5.29(2H, ABq, J=12.2Hz), 7.12(1H, dd, J=1.7Hz, 7.9Hz), 7.25(1H, t, J=7.9Hz), 7.33-7.51(8H, m), 7.55-7.63(2H, m), 8.07(1H, dd, J=1.7Hz, 7.9Hz)

Preparation 15-(1)

1-ethoxycarbonylmethyl-5-oxo-6-hydroxy-8-(2-fluorophenyl) -2, 3, 5, 6-tetrahydro-1H-[1, 4] diazepino [1, 7, 6-de] quinoxaline was obtained according to a similar manner to that of preparation 14.

mp: 175-178 °C
IR(Nujol):3600, 1710, 1690, 1600, 1670$cm^{-1}$
NMR($CDCl_3$)$\delta$:3.43-3.56(1H, m), 3.65-3.82(1H, m), 4.32(1H, quIntet, J=6.5Hz), 4.61 (1H, quIntet, J=6.5Hz), 5.05(1H, s), 5.29(2H, ABq, J=12.2Hz), 7.12(1H, dd, J=1.7Hz, 7.9Hz), 7.25(1H, t, J=7.9Hz), 7.33-7.51(8H,

m), 7.55-7.63(2H, m), 8.07(1H, dd, J=1.7Hz, 7.9Hz)

Preparation 15-(2)

1-methoxycarbonyl-5-oxo-6-hydroxy-8-phenyl-2, 3, 5, 6-tetrahydro-1H-[1, 4]diazepino[1, 7, 6-de]-quinoxaline was obtained according to a similar manner to that of preparation 14.
IR(Nujol):3400, 1690, 1610, 1575cm$^{-1}$
NMR($CDCl_3$)δ:3.46-3.58(1H, m), 3.65-3.77(1H, m), 3.87(3H, s), 4.25-4.35(1H, m), 4.40-4.80(2H, m), 5.04(1H, s), 7.13(1H, dd, J=1.7Hz, 7.9Hz), 7.22(1H, t, J=7.9Hz), 7.33-7.51(3H, m), 7.58-7.63(2H, m), 8.30(1H, dd, J=1.4Hz, 7.9Hz)

Preparation 16

To a solution of 1-ethoxycarbonylmethyl-5-oxo-6-hydroxy -8-(2-fluorophenyl)-2, 3, 5, 6-tetrahydro-1H-[1, 4] diazepino [1, 7, 6-de]quinoxaline (1.223g) and triethylamine (622.8mg) in dichloromethane (15ml) was added dropwise a solution of methanesulfonyl chloride (0.36ml) in dichloromethane (2ml) under stirring at 2~3°C cooling in an ice-bath. The mixture was stirred for 1.5 hours under the same conditions. The reaction mixture was poured into a solution of 28% aqueous ammonia (10ml) in acetonitrile (10ml) under stirring at 30°C. The mixture was stirred under the same conditions for 1 hour. The reaction mixture was evaporated in vacuo and the residue was extracted twice with ethyl acetate. The combined extract was washed with water, dried over magnesium sulfate and evaporated in vacuo. The residue was subjected to column chromatography on silica gel eluting with chloroform, a mixture of chloroform and methanol (50:1) successively to give pure 1-ethoxycarbonylmethyl-5-oxo-6 -amino-8-(2-fluorophenyl)-2, 3, 5, 6-tetrahydro-1H-[1, 4] diazepino [1, 7, 6-de]quinoxaline (0.91g) as an orange amorphous mass.
Mass(m/e):396($M^+$)
IR(Film):3360, 3300, 1740, 1678, 1610, 745cm$^{-1}$
NMR($CDCl_3$)δ:1.27(3H, t, J=7.1Hz), 2.39(2H, brood s), 3.10(1H, dt, J=3.5Hz, 12.0Hz) 3.52(1H, td, J=1.7Hz, 7.7Hz), 3.71(1H, dt, J=4.0Hz, 11.4Hz), 4.11(2H, dd, J=18.1Hz, 46.0Hz), 4.22(2H, q, J=7.1Hz), 4.62(1H, s), 5.00(1H, td, J=2.3Hz, 11.1Hz)

Preparation 17-(1)

1-methoxycarbonyl-5-oxo-6-amino-8-phenyl-2, 3, 5, 6-tetrahydro-1H-[1, 4]diazepino[1, 7, 6-de]-quinoxaline was obtained according to a similar manner to that of preparation 16.
NMR(DMSO)δ:3.20-4.00(2H, m), 3.30-3.50(1H, m), 3.60-3.80 (1H, m), 3.76(3H, s), 4.0-4.20(1H, m), 4.30-4.50-(1H, m), 4.56(1H, s), 7.04(1H, d, J=6.7Hz), 7.28(1H, t, J=8.0Hz), 7.40-7.70(5H, m), 8.03(1H, d, J=8.0Hz)

Preparation 17-(2)

1-benzyloxycarbonyl-5-oxo-6-amino-8-phenyl-2, 3, 5, 6-tetrahydro-1H-[1, 4]diazepino[1, 7, 6-de]-quinoxaline was obtained according to a similar manner to that of preparation 16. NMR($CDCl_3$)δ:2.17(2H, s), 3.34-3.50(1H, m), 3.69-3.82(1H, m), 4.28(1H, quIntet, J=5.6Hz), 4.55(1H, s), 4.62(1H, quIntet, J=5.6Hz), 5.28(2H, ABq, J=12.2Hz), 7.09(1H, dd, J=1.8Hz, 7.9Hz), 7.21(1H, t, J=7.9Hz), 7.34-7.50(8H, m), 7.56-7.61-(2H, m), 8.05(1H, d, J=7.9Hz)

Example 1

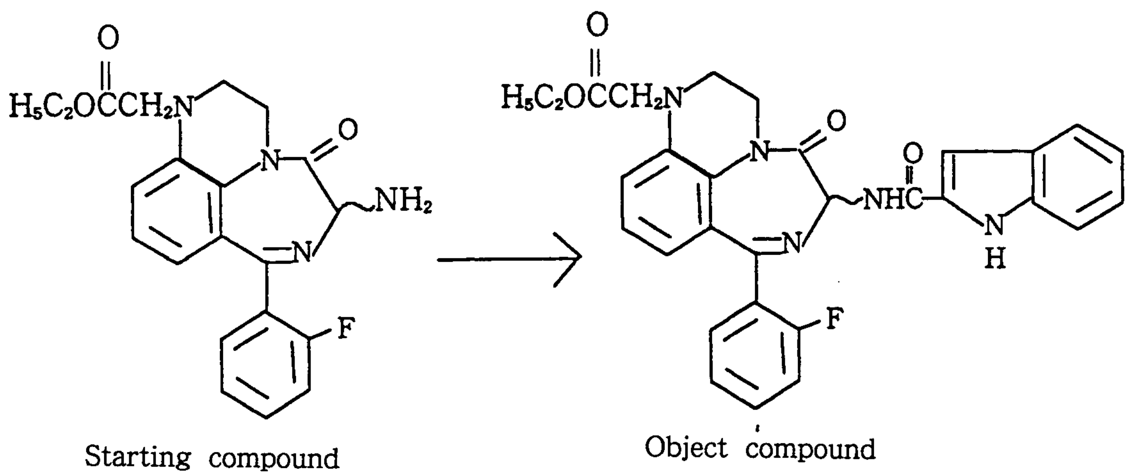

To a solution of 1-ethoxycarbonylmethyl-5-oxo-6-amino-8 -(2-fluorophenyl)-2, 3, 5, 6-tetrahydro-1H-[1, 4] diazepino [1, 7, 6-de]quinoxaline (0.27g) and indole-2-carboxylic acid (0.13g) in dimethylformamide (3ml) were added successively N-hydroxybenzotriazole (0.11g), 1 - ethyl - 3 - ( 3 - dimethylamino) propylcarbodiimide hydrochloride (0.16g) and triethylamine (83mg) under stirring at ambient temperature. The mixtuer was stirred for 15 hours under the same conditions. To the reaction mixture were added successively ethyl acetate, water and a diluted aqueous solution of sodium bicarbonate to adjust pH 8.0. The resultant mixture was stirred vigorously for 0.5 hour. The separated organic layer was washed with water, dried over magnesium sulfate and evaporated in vacuo. The residue was subjected to column chromatography on silica gel eluting with chloroform to afford an oil, which was triturated in diisopropyl ether to give 1 - ethoxycarbonylmethyl - 5 - oxo - 6 - ( 2 - indolylcarbonylamino)-8-(2-fluorophenyl)-2, 3, 5, 6-tetrahydro-1H-[1, 4]diazepino [1, 7, 6-de]quinoxaline (279.0mg) as a yellow powder.
Mass(m/e):539($M^+$)
mp: 243-248 ° C(dec.)
IR(Nujol):3340, 1726, 1665(sh), 1650, 743$cm^{-1}$
NMR(DMSO-$d_6$)δ:1.21(3H, t, J=7.1Hz), 3.03(1H, br. t, J=9.9Hz), 3.51(2H, br. t, J=10.9Hz), 4.15(2H, q, J=7.1Hz), 4.36(2H, ABq, J=18.3Hz, 39.3Hz), 4.81(1H, br. d, J=12.2Hz), 5.72(1H, d, J=8.1Hz), 6.47-7.67-(12H, m), 9.62(1H, d, J=8.1Hz), 11.66(1H, s)

Example 2-(1)

1-benzyloxycarbonyl-5-oxo-6-(2-indolylcarbonylamino)-8-phenyl-2, 3, 5, 6-tetrahydro-1H-[1, 4]diazepino-[1, 7, 6-de] quinoxaline was obtained according to a similar manner to that of Example 1.
mp: 202-206 ° C
IR(Nujol):3290, 1720, 1695, 1640, 1540$cm^{-1}$
NMR(DMSO-$d_6$)δ:3.70-3.46(1H, m), 3.75-3.95(1H, m), 4.37-4.57(1H, m), 5.26(2H, ABq, J=12.7Hz), 5.69(1H, d, J=7.9Hz), 7.02-7.68(17H, m), 8.15(1H, d, J=7.6Hz), 9.54(1H, d, J=7.9Hz), 11.65(1H, s)

Example 2-(2)

1-methoxycarbonyl-5-oxo-6-(2-indolylcarbonylamino)-8-phenyl-2, 3, 5, 6-tetrahydro-1H-[1, 4]diazepino[1, 7, 6-de] quinoxaline was obtained according to a similar manner to that of Example 1.
mp: 286-288 ° C
IR(Nujol):3340, 3260, 1700, 1630, 1540$cm^{-1}$
NMR(DMSO)δ:3.35-3.55(1H, m), 3.70-3.85(1H, m), 3.78(3H, s), 4.05-4.20(1H, m), 4.38-4.50(1H, m), 5.68(1H, d, J=8.0Hz), 7.02 -7.67(12H, m), 8.10(1H, d, J=8.0Hz), 9.55(1H, d, J=8.0Hz), 11.65 (1H, s)

Example 3

To a solution of 1-ethoxycarbonylmethyl-5-oxo-6-amino-8 -(2-fluorophenyl)-2, 3, 5, 6-tetrahydro-1H-[1, 4] diazepino [1, 7, 6-de]quinoxaline (0.18g) in dry tetrahydrofuran (2ml) was added 4-chlorophenyl isocyanate (70mg) under stirring at ambient temperatuer and the mixture was stirred for 1.5 hours. To the reaction mixture was added diisopropyl ether (15ml) and the mixture was cooled in an ice-bath for 0.5 hour under stirring. The resultant precipitate was collected by filtration and washed with diisopropyl ether to give pure 1-ethoxycarbonylmethyl-5-oxo -6-(3-(4-chlorophenyl)ureido)-8-(2-fluorophenyl)-2, 3, 5, 6-tetrahydro-1H-[1, 4]-diazepino[1, 7, 6-de]quinoxaline (198.3mg) as white powder.
Mass(m/e): 549($M^+$), 550($M^+$ +1), 551($M^+$ +1)
mp: 259-261 °C
IR(Nujol):3300, 1728, 1670, 1639, 1595$cm^{-1}$
NMR(DMSO-$d_6$)δ:1.19(3H, t, J=7.1Hz), 3.02(1H, m), 3.54(2H, m), 4.15(2H, q, J=7.1Hz), 4.35(2H, ABq, J=18.3Hz, 40.7Hz), 4.79(1H, d, J=12.4Hz), 5.33(1H, d, J=8.4Hz), 6.46(1H, d, J=7.5Hz), 6.86(1H, d, J=7.5Hz), 7.07(1H, t, J=7.5Hz), 7.17-7.6(9H, m), 9.19(1H, s)

Example 4

1-benzyloxycarbonyl-5-oxo-6-(3-(4-chlorophenyl)ureido)-8-phenyl-2, 3, 5, 6-tetrahydro-1H-[1, 4]-diazepino[1, 7, 6-de] quinoxaline was obtained according to a similar manner to that of Example 3.
mp: 217- 218 °C
IR(Nujol):3310, 1700, 1675, 1640, 1600, 1540$cm^{-1}$
NMR(DMSO)δ:3.30-3.50(1H, m), 3.75-3.90(1H, m), 4.05-4.25 (1H, m), 4.40-4.55(1H, m), 5.24(1H, ABq, J=12.5Hz), 5.33(1H, d, J=8.1Hz), 7.07(1H, dd, J=1.3Hz, 7.8Hz), 7.25-7.58(16H, m), 8.13 (1H, d, J=7.1Hz), 9.21(1H, s)

Example 5

To a suspension of 1-ethoxycarbonylmethyl-5-oxo-6-(3-(4 -chlorophenyl)ureido)-8-(2-fluorophenyl)-2, 3, 5, 6-tetrahydro-1H -[1, 4]diazepino[1, 7, 6-de]quinoxaline (660mg) in ethanol (35ml) was added 0.1N aqueous solution of sodium hydroxide (13.2ml) under stirring at ambient temperature. The mixtuer was warmed at 80 °C under stirring for 1 hour. Ethanol was removed in vacuo to givea yellow aqueous solution, which was made acidic with dil. aqueous HCl to precipitate. The resultant precipitate was collected by filtration, washed with water several times and dried over diphosphorus pentaoxide in a desicator under reduced pressure to give pure 1 - carboxymethyl-5-oxo-6-(3-(4-chlorophenyl)ureido)-8-(2-fluorophenyl)-2, 3, 5, 6-tetrahydro-1H -[1, 4]diazepino[1, 7, 6-de]quinoxaline (606.1mg) as a white powder.
Mass(m/e):477($M^+$ -44), 478[($M^+$ +1)-44], 479[($M^+$ +2)-44], 450 [($M^+$ +3)-44]
mp: 227-232 °C(dec.)
IR(Nujol):3270, 2800-2300(brood), 1740, 1665, 1638, 820, 750$cm^{-1}$
NMR(DMSO-$d_6$)δ:3.0(1H, br. t), 3.55(2H, br. s), 4.12(2H, ABq, J=18.1Hz, 38.9Hz), 4.75(1H, br. d), 5.33(1H, d, J=8.4Hz), 6.41(1H, d, J=7.3Hz), 6.8-7.6(11H, m), 9.24(1H, s)

Example 6

1-carboxymethyl-5-oxo-6-(2-indolylcarbonylamino)-8-(2-fluorophenyl)-2, 3, 5, 6-tetrahydro-1H-[1, 4]-diazepino[1, 7, 6 -de]quinoxaline was obtained according to a similar manner to that of Example 5.
IR(Nujol):3300, 2700-2300(brood), 1718, 1690, 1635, 770, 742$cm^{-1}$
NMR(DMSO-$d_6$)δ:3.02(1H, br. t), 3.56(2H, br. s), 4.24(2H, ABq, J=18.4Hz, 35.6Hz), 4.80(1H, br. d, J=12.5Hz), 5.71 (1H, d, J=8.1Hz), 6.47(1H, d, J=7.4Hz), 6.86(1H, d, J=7.4Hz), 7.0-7.7(10H, m), 9.61 (1H, d, J=8.1Hz), 11.66(1H, s), 12.6-13.2(1H, brood)

Example 7

To a suspended solution of 1-(3-methoxycarbonylpropionyl) -5-oxo-6-(2-indolylcarbonylamino)-8-phenyl-2, 3, 5, 6-tetrahydro -1H-[1, 4]diazepino[1, 7, 6-de]quinoxaline (2.17g) in a mixed solvent of dioxane (65ml) and methanol (22ml) was added 0.5N aqueous solution of lithium hydroxide (7.9ml) under stirring at ambient temperature. After stirring for 18 hours under the same conditions, the rection mixture was heated at 50 °C under stirring for 2.5 hours. An additional 0.5N aqueous solution of lithium hydroxide (3ml) was

added to the rection mixture at ambient temperature and the mixture was stirred for 16 hours at the same temperature. The mixture was neutralized with 1N HCl aqueous solution (5.45ml) and evaporated in vacuo. After the residue was partitioned between chloroform and water, the insoluble precipitate was filtered off. The separated organic layer was evaporated in vacuo. The residue was subjected to column chromatography on silica gel (200ml) eluting with a mixture of dichloromethane and methanol (50 : 1 by volume). The desired fractions were combined and evaporated in vacuo. The residue was triturated in diethyl ether and the resultant precipitate was collected by filtration to give 1-(3-carboxypropionyl)-5-oxo-6-(2 -indolylcarbonylamino)-8-phenyl-2, 3, 5, 6-tetrahydro-1H-[1, 4] diazepino[1, 7, 6-de]quinoxaline (90mg).
mp: 185-195 ° C
IR(Nujol):3250, 1640, 1530cm$^{-1}$
NMR(DMSO)δ:2.60-3.80(6H, m), 4.0-4.30(1H, m), 4.50-4.70(1H, m), 5.60-5.75(1H, m), 7.02-7.68(13H, m), 7.95(1H, d, J = 7.2Hz), 9.56(1H, d, J = 7.9Hz), 11.73(1H, s)

Example 8

A mixture of 1-[3-[((2S)-2-benzyloxycarbonylpyrrolidin-1-yl)carbonyl]propionyl]-5-oxo-6-(2-indolylcarbonylamino)-8-phenyl -2, 3, 5, 6-tetrahydro-1H-[1, 4]diazepino[1, 7, 6-de]quinoxaline (65mg), 10% palladium-charcoal (30mg) and tetrahydrofuran (4ml) was subjected to hydrogenation reaction under hydrogen atmosphere (1 atm.) at ambient temperature for 10 hours with stirring. The catalyst was filtered off and the filtrate was evaporated in vacuo. The residue was suspended in diethy ether and the resultant crystal was collected by filtration to give 1-[3 -[((2S)-2-carboxypyrrolidin-1-yl)carbonyl]propionyl]-5-oxo-6-(2-indolylcarbonylamino)-8-phenyl-2, 3, 5, 6-tetrahydro-1H-[1, 4] diazepino[1, 7, 6-de]quinoxaline (47mg).
mp: 184-192 ° C
IR(Nujol):3250, 1630, 1530cm$^{-1}$
NMR($CDCl_3$)δ:1.15-1.35(2H, m), 1.80-2.05(3H, m), 3.05-3.30 (1H, m), 3.40-3.80(2H, m), 4.20-4.55(2H, m), 4.80-5.10(1H, m), 5.85-5.95(1H, m), 7.0-7.80(14H, m), 8.05-8.20(1H, m), 9.60-9.85(1H,m)

Example 9

To a solution of 1-carboxymethyl-5-oxo-6-(3-(4-chlorophenyl)ureido)-8-(2-fluorophenyl)-2, 3, 5, 6-tetrahydro-1H -[1, 4]diazepino[1, 7, 6-de]quinoxaline (150mg) in dimethylformamide (2ml) were added successively 1 - methylpiperazine (34.6mg), N-hydroxybenzotriazole (46.7mg), 1-ethyl-3-(3-dimethylamino)-propylcarbodiimide hydrochloride (66.3mg) and triethylamine (35.0mg) under stirring at ambient temperature. The mixtuer was stirred for 19 hours under the same conditions. The rection mixture was poured into water under stirring and the resultant precipitate was collected by filtration, washed with water and air-dried. The obtained powder (148.5mg) was subjected to column chromatography on silica gel eluting with a mixture of chloroform and methanol (50:1~30:1). The fractions containing the desired compound were combined and evaporated in vacuo. The residue was triturated in diisopropyl ether and collected by filtration to give 1-[(4-methylpiperazin-1-yl)carbonylmetyl]-5-oxo-6-(3-(4-chlorophenyl) ureido)-8-(2-fluorophenyl)-2, 3, 5, 6-tetrahydro-1H-[1, 4] diazepino[1, 7, 6-de]quinoxaline (40.1mg) as white powder.
FAB Mass(m/e): 604($M^+$), 605($M^+$ + 1), 606($M^+$ + 2)
mp: 173-177 ° C(dec.)
IR(Nujol): 3320, 1660cm$^{-1}$
NMR(DMSO-$d_6$)δ:2.27-2.38(8H, m), 3.02(1H, m), 3.34-3.6(5H, m), 4.40(2H, ABq, J = 17.8Hz, 43.6Hz), 4.77-(1H, d, J = 12.0Hz), 5.33 (1H, J = 8.4Hz), 6.39(1H, d, J = 7.4Hz), 6.76-7.57(11H, m), 9.20(1H, s)

Example 10-(1)

1-(1-pyrrolidinylcarbonylmethyl)-5-oxo-6-(3-(4-chlorophenyl)ureido)-8-(2-fluorophenyl)-2, 3, 5, 6-tetrahydro-1H -[1, 4]diazepino[1, 7, 6-de]quinoxaline was obtained according to a similar manner to that of Example 9.
Mass(m/e):463($M^+$ -112)
mp: 179-182 ° C(dec.)
IR(Nujol):3325, 1675, 1655, 1610cm$^{-1}$
NMR(DMSO-$d_6$)δ:1.59(4H, br. s), 2.50(4H, br. s), 3.02(1H, br. t), 3.43(2H, br. s), 4.39(2H, ABq, J = 17.5Hz, 42.6Hz), 4.77(1H, br. d, J = 12.3Hz), 5.33(1H, d, J = 8.3Hz), 6.39(1H, d, J = 7.5Hz), 6.76(1H, d, J = 7.5Hz), 7.03(1H, t, J = 7.5Hz), 7.17-7.58(9H, m), 9.21(1H, s)

Example 10-(2)

1-[3-[(4-methylpiperazin-1-yl)carbonyl]propionyl]-5-oxo-6 -(2-indolylcarbonylamino)-8-phenyl-2, 3, 5, 6-tetrahydro-1H-[1, 4]diazepino[1, 7, 6-de]quinoxaline was obtained according to a similar manner to that of Example 9.
FAB Mass:618($M^+$ +1)
mp: 175-185 ° C
IR(Nujol):3250, 1630, 1530$cm^{-1}$
NMR(DMSO)δ:1.85-2.0(3H, m), 2.04(3H, s), 2.10-2.35(3H, m), 2.55-2.75(4H, m), 3.55-3.70(1H, m), 3.95-4.20-(1H, m), 4.60-4.80 (1H, m), 5.67(1H, d, J=8.2Hz), 7.0-7.95(13H, m), 9.40(1H, d, J=8.0Hz), 11.68(1H, s)

Example 10-(3)

1-[3-[((2S)-2-benzyloxycarbonylpyrrolidin-1-yl)carbonyl] propionyl]-5-oxo-6-(2-indolylcarbonylamino)-8-phenyl-2, 3, 5, 6 -tetrahydro-1H-[1, 4]diazepino[1, 7, 6-de]quinoxaline was obtained according to a similar manner to that of Example 9.
FAB Mass:723($M^+$ +1)
mp: 156-165 ° C
IR(Nujol):3250, 1730, 1630, 1530$cm^{-1}$
NMR($CDCl_3$)δ:1.15-1.45(2H, m), 1.80-2.20(4H, m), 2.45-2.85 (6H, m), 3.05-3.4(2H, m), 3.60-3.95(1H, m), 4.30-4.95(3H, m), 6.55-6.60(1H, m), 6.75-7.75(15H, m), 8.25-8.40(1H, m), 8.41(1H, d, J=4.2Hz), 8.50-8.65-(1H, m)

Example 11

A mixture of 1-benzyloxycarbonyl-5-oxo-6-(2-indolylcarbonylamino)-8-phenyl-2, 3, 5, 6-tetrahydro-1H-[1, 4] diazepino[1, 7, 6-de]quinoxaline (0.13g), ethyl acetate (5ml), methanol (7.5ml) and 10% palladium - charcoal as catalyst was subjected to hydrogenation reaction under hydrogen atmosphere (1 atm.) at ambient temperature for 1 hour with stirring. The catalyst was filtered off and the filtrate was evaporated in vacuo. The residue was suspended in ethyl acetate and the resultant precipitate was collected by filtration to give 5-oxo-6-(2-indolylcarbonylamino)-8-phenyl-2, 3, 5, 6-tetrahydro-1H-[1, 4] diazepino[1, 7, 6-de]-quinoxaline (99mg).
IR(Nujol):3340, 3280, 1660, 1640, 1610, 1590, 1540, 1510$cm^{-1}$
NMR(DMSO-$d_6$)δ:2.80-3.00(1H, m), 3.15-3.30(1H, m), 3.50-3.65(1H, m), 4.65-4.75(1H, m), 5.73(1H, d, J=8.1Hz), 6.47(1H, d, J=6.4Hz), 6.75-6.81(1H, m), 6.89(1H, d, J=6.8Hz), 7.06(2H, t, J=7.7Hz), 7.21(1H, t, J=6.9Hz), 7.40-7.62(8H, m), 9.52(1H, d, J=8.1Hz), 11.65(1H, s)

Example 12

5-Oxo-6-(3-(4-chlorophenyl)ureido)-8-phenyl-2, 3, 5, 6-tetrahydro-1H-[1, 4]diazepino[1, 7, 6-de]-quinoxaline was obtained according to a similar manner to that of Example 11.
mp: 270-272 ° C(dec.)
IR(Nujol):3300, 1670, 1630, 1590, 1545$cm^{-1}$
NMR(DMSO)δ:2.80-2.95(1H, m), 3.10-3.30(1H, m), 3.45-3.60 (1H, m), 4.60-4.75(1H, m), 5.36(1H, d, J=8.3Hz), 6.41-6.45(1H, m), 6.70-6.80(1H, m), 6.84-6.88(1H, m), 7.04(1H, t, J=7.9Hz), 7.15-7.53(10H, m)

Example 13

A mixture of fumaric acid monoethyl ester (20mg), thionyl chloride (0.02ml) and dry dimethylformamide (3mg) in dry dichloromethane was refluxed under stirring for 3 hours. The obtained acid chloride solution was added dropwise to a mixture of 5-oxo-6-(2-indolylcarbonylamino)-8-phenyl-2, 3, 5, 6-tetrahydro-1H-[1, 4]diazepino[1, 7, 6-de]quinoxaline (47mg) and N, O-bis(trimethylsilyl)acetamide (0.13ml) in dry dioxane (1.5ml) under stirring at ambient temperature. After stirring for 2 hours under the same conditions, the mixture was evaporated in vacuo. The residue was partitioned between dichloromethane and water. The separated organic layer was washed with water and brine successively, dried over magnesium sulfate and evaporated in vacuo. The residue was subjected to column chromatography on silica gel (20ml) eluting with a mixture of dichloromethane and methanol (100:1 by volume). The fractions containing the desired compound were combined and evaporated in vacuo. The residue was suspended in diethyl ether and the

resultant precipitate was collected by filtration to give 1-((E)-3-ethoxycarbonylacryloyl)-5 -oxo-6-(2-indolylcarbonylamino)-8-phenyl-2, 3, 5, 6-tetrahydro-1H-[1, 4]diazepino[1, 7, 6-de]quinoxaline (22mg).
mp: 219-221 ° C
IR(Nujol):3350, 3275, 1710, 1670, 1630, 1540cm$^{-1}$
NMR(DMSO)δ:1.22(3H, t, J = 7.1Hz), 3.33-3.59(1H, m), 3.60-3.80 (1H, m), 4.10-4.30(1H, m), 4.19(2H, q, J = 7.1Hz), 5.74(1H, d, J = 8.0Hz), 6.73(1H, d, J = 15.5Hz), 7.05-7.68(14H, m), 9.43(1H, d, J = 8,0Hz), 11.69-(1H, s)

Example 14

3-Carbomethoxypropionyl chloride (2.26ml) was added to a mixture of 5-oxo-6-(2-indolylcarbonylamino)-8-phenyl-2, 3, 5, 6-tetrahydro-1H-[1, 4]diazepino[1, 7, 6-de]quinoxaline (2.0g) and pyridine (1.6ml) in tetrahydrofuran (80ml) under stirring at ambient temperature. After stirring for 22 hours under the same conditions, the reaction mixture was evaporated in vacuo. The residue was suspended in water and the resultant precipitate was collected by filtration. Then the collected precipitate was suspended in methanol and collected by filtration to give 1-(3-methoxycarbonylpropionyl)-5-oxo-6-(2-indolylcarbonylamino)-8-phenyl-2, 3, 5, 6-tetrahydro-1H-[1, 4]diazepino[1, 7, 6-de] quinoxaline (2.25g).
mp: 236-237 ° C
IR(Nujol):3350, 3280, 1730, 1675, 1655, 1630, 1540cm$^{-1}$
NMR(DMSO)δ:2.50-3.10(4H, m), 3.20-3.8(2H, m), 3.56(3H, s), 4.05-4.30(1H, m), 4.0-4.75(1H, m), 5.69(1H, d, J = 8.1Hz), 7.06(1H, t, J = 7.0Hz), 7.18-7.68(11H, m), 7.93(1H, d, J = 7.3Hz), 9.53(1H, d, J = 8.1Hz), 11.67(1H, s)

Example 15

1-(3-methoxycarbonylpropionyl)-5-oxo-6-(3-(4-chlorophenyl)ureido)-8-phenyl-2, 3, 5, 6-tetrahydro-1H-[1, 4] diazepino[1, 7, 6- de]quinoxaline was obtained according to a similar manner to that of Example 14.
mp: 246-248 ° C(dec.)
IR(Nujol):3340, 1740, 1675, 1645, 1605, 1545cm$^{-1}$
NMR(DMSO)δ:2.50-3.00(4H, m), 3.10-3.70(2H, m), 3.54(3H, s), 5.21(1H, d, J = 8.1Hz), 7.18-7.61(12H, m), 7.88(1H, d, J = 8.3Hz), 9.23(1H, s)

## Claims

**1.** A compound of the formula:

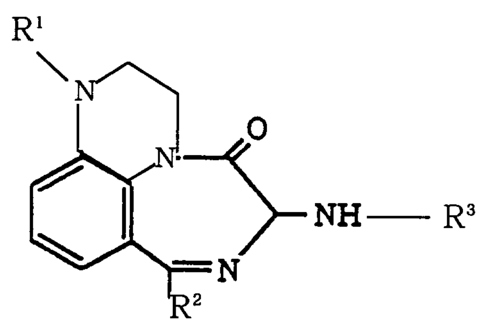

wherein $R^1$ is hydrogen, carboxy, protected carboxy, carboxy (lower) alkyl, protected carboxy (lower) alkyl, carboxy (lower) alkanoyl, protected carboxy (lower) alkanoyl, carboxy (lower) alkenoyl, protected carboxy (lower) alkenoyl, heterocycliccarbonyl (lower) alkyl which may have suitable substituent (s), or heterocycliccarbonyl (lower) alkanoyl which may have suitable substituent (s), $R^2$ is aryl which may have suitable substituent (s), $R^3$ is acyl, and a pharmaceutically acceptable salt thereof.

**2.** A compound of claim 1,
wherein $R^1$ is hydrogen, carboxy, protected carboxy, carboxy (lower) alkyl, protected carboxy

(lower) alkyl,carboxy (lower) alkanoyl, protected carboxy (lower) alkanoyl, carboxy (lower) alkenoyl,protected carboxy (lower) alkenoyl,heterocycliccarbonyl (lower) alkyl which may have 1 to 3 suitable substituent (s), or heterocycliccarbonyl (lower) alkanoyl which may have 1 to 3 suitable substituent (s), and

$R^2$ is aryl which may have 1 to 3 suitable substituent (s).

3. A compound of claim 2,
wherein $R^1$ is hydrogen,carboxy, esterified carboxy, carboxy (lower) alkyl, esterified carboxy (lower) alkyl, carboxy (lower) alkanoyl, esterified carboxy (lower) alkanoyl, carboxy (lower) alkenoyl, esterified carboxy (lower) alkenoyl, heterocycliccarbonyl (lower) alkyl which may have lower alkyl, or heterocycliccarbonyl (lower) alkanoyl which may have substituent (s) selected from the group consisting of lower alkyl, carboxy and protected carboxy,
$R^2$ is phenyl which may have one suitable substituent, and
$R^3$ is heterocyclic carbonyl; or arylcarbamoyl which may have 1 to 3 suitable substituent (s).

4. A compound of claim 3,
wherein $R^1$ is hydrogen,carboxy,lower alkoxycarbonyl, mono (or di or tri) phenyl (lower) alkoxycarbonyl, carboxy (lower) alkyl, lower alkoxycarbonyl (lower) alkyl, carboxy (lower) alkanoyl, lower alkoxycarbonyl (lower) alkanoyl, carboxy (lower) alkenoyl, lower alkoxycarbonyl (lower) alkenoyl, pyrrolidinylcarbonyl (lower) alkyl, lower alkylpiperazinylcarbonyl ( lower ) alkyl , lower alkylpiperazinylcarbonyl ( lower ) alkanoyl , carboxypyrrolidinylcarbonyl (lower) alkanoyl or protected carboxypyrrolidinylcarbonyl (lower) alkanoyl,
$R^2$ is phenyl or halophenyl, and
$R^3$ is indolylcarbonyl, or phenylcarbamoyl which may have one suitable substituent.

5. A compound of claim 4,
wherein $R^3$ is indolylcarbonyl or halophenylcarbamoyl.

6. A compound of claim 5,
wherein $R^1$ is carboxypyrrolidinylcarbonyl (lower) alkanoyl,
$R^2$ is phenyl,and
$R^3$ is indolylcarbonyl.

7. A compound of claim 6 which is 1 - [3 - [((2S) - 2 - carboxypyrrolidin - 1 - yl) carbonyl] propionyl] - 5 - oxo - 6 - (2 - indolylcarbonylamino) - 8 - phenyl - 2,3,5,6 - tetrahydro - 1H - [1,4] diazepino [1,7,6-de] quinoxaline.

8. A process for the preparation of a compound of the formula :

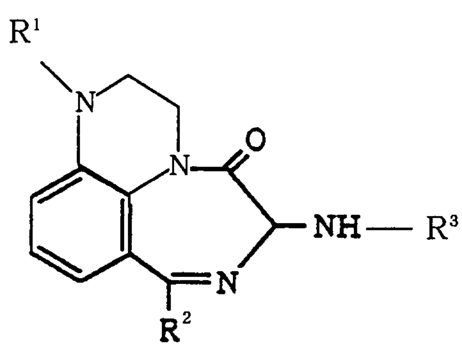

wherein $R^1$, $R^2$ and $R^3$ are each as defined below, or a salt thereof, which comprises
i) subjecting a compound of the formula:

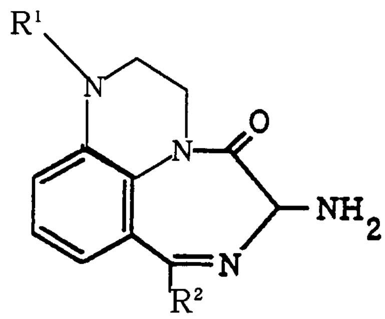

wherein $R^1$ is hydrogen,carboxy,protected carboxy, carboxy (lower) alkyl, protected carboxy (lower) alkyl, carboxy (lower) alkanoyl, protected carboxy (lower) alkanoyl, carboxy (lower) alkenoyl, protected carboxy (lower) alkenoyl, heterocycliccarbonyl (lower) alkyl which may have suitable substituent (s), or heterocycliccarbonyl (lower) alkanoyl which may have suitable substituent (s),

$R^2$ is aryl which may have suitable substituent (s), or its reactive derivative at the amino group or a salt thereof,

to acylation reaction,to give a compound of the formula :

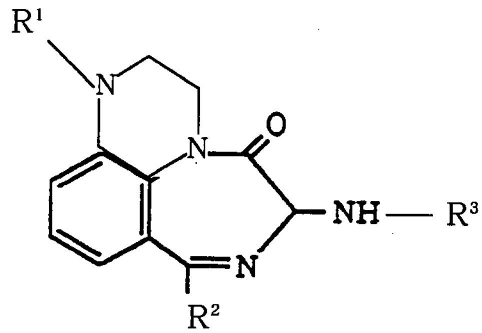

wherein $R^1$ and $R^2$ are each as defined above, and
$R^3$ is acyl, or a salt thereof, or
ii) subjecting a compound of the formula:

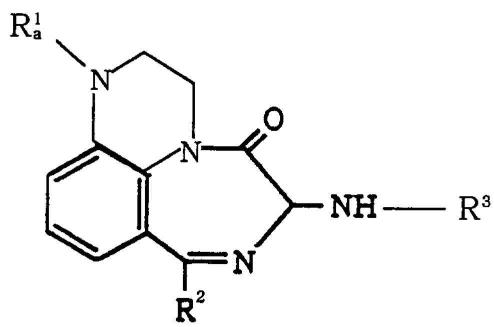

wherein $R^2$ and $R^3$ are each as defined above, and

$R^1_a$ is protected carboxy, protected carboxy (lower) alkyl, protected carboxy (lower) alkanoyl, protected carboxy (lower) alkenoyl, or heterocycliccarbonyl (lower) alkanoyl which has protected carboxy, or a salt thereof, to elimination reaction of the carboxy - protective group,

to give a compound of the formula:

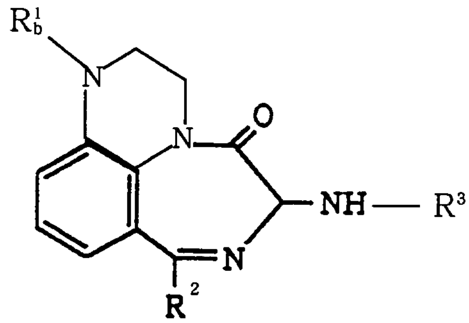

wherein $R^2$ and $R^3$ are each as defined above, and

$R_b^1$ is carboxy , carboxy (lower) alkyl, carboxy (lower) alkanoyl, carboxy (lower) alkenoyl, or heterocycliccarbonyl (lower) alkanoyl which has carboxy, or a salt thereof, or

iii) reacting a compound of the formula:

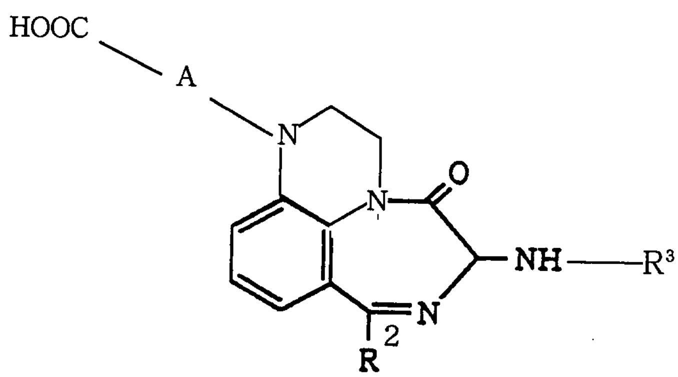

wherein $R^2$ and $R^3$ are each as defined above, and

A is lower alkylene, or its reactive derivative at the carboxy group or a salt thereof, with a compound of the formula :

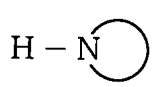

wherein

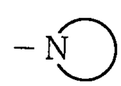

is piperazinyl or pyrrolidinyl,

each of which may have suitable substituent (s) to give a compound of the formula:

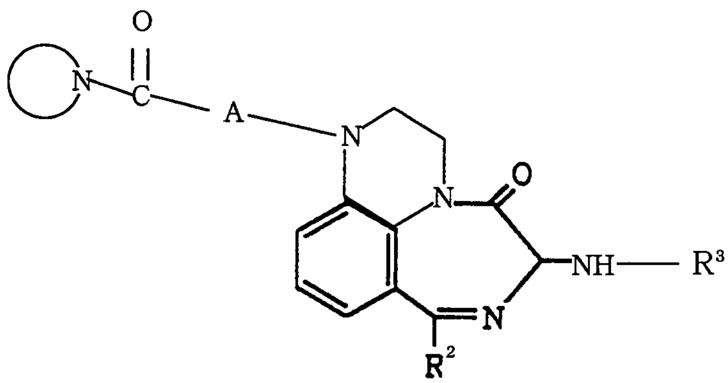

wherein $R^2$,$R^3$,A and

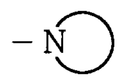

are each as defined above, or a salt thereof, or
iv) reacting a compound of the formula:

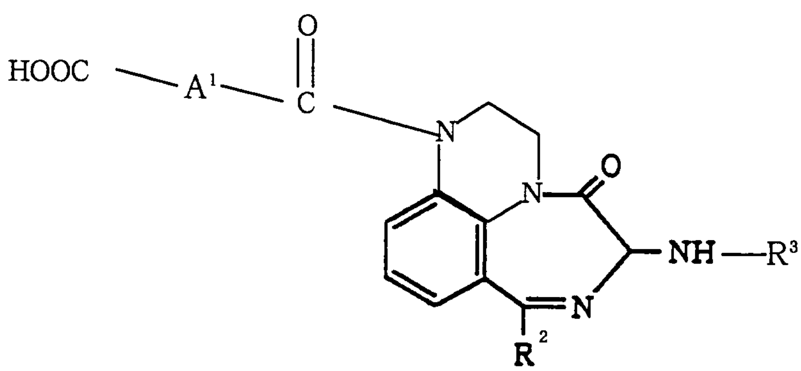

wherein $R^2$ and $R^3$ are each as defined above, and

$A^1$ is ($C_1$ - $C_5$) alkylene, or its reactive derivative at the carboxy group or a salt thereof, with a compound of the formula :

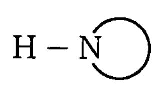

wherein

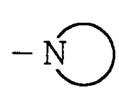

is as defined above, or a salt thereof, to give a compound of the formula:

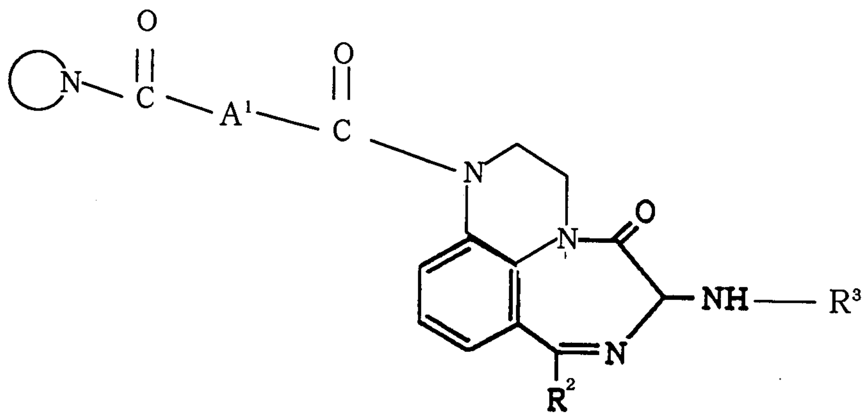

wherein $R^2$,$R^3$,$A^1$ and

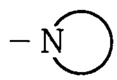

are each as defined above, or a salt thereof, or
v) subjecting a compound of the formula:

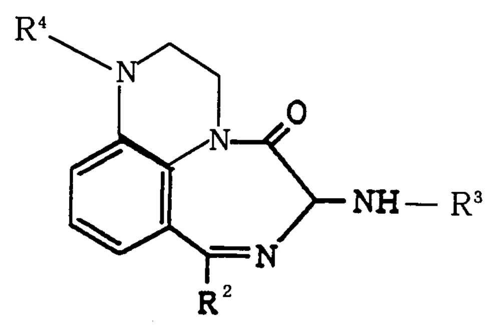

wherein $R^2$ and $R^3$ are each as defined above, and
$R^4$ is imino - protective group, or a salt thereof, to elimination reaction of the imino - protective group,
to give a compound of the formula:

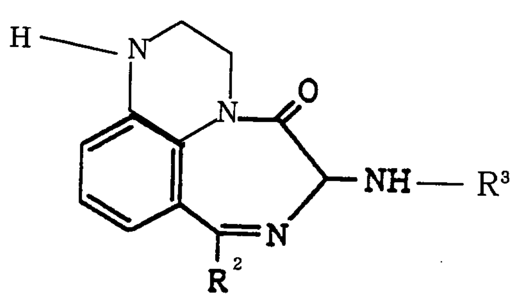

wherein $R^2$ and $R^3$ are each as defined above, or a salt thereof, or

vi) reacting a compound of the formula:

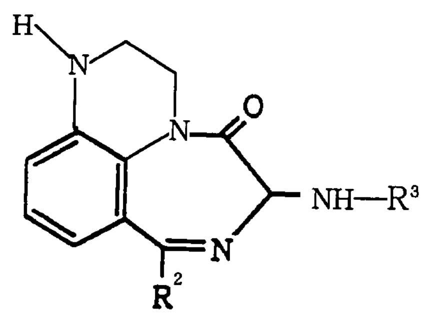

wherein $R^2$ and $R^3$ are each as defined above,
or a salt thereof, with a compound of the formula :

$R_c^1$ - OH

wherein $R_c^1$ is carboxy (lower) alkanoyl, protected carboxy (lower) alkanoyl, carboxy (lower) alkenoyl, protected carboxy (lower) alkenoyl, or heterocycliccarbonyl (lower) alkanoyl which may have suitable substituent (s), or its reactive derivative at the carboxy group
or a salt thereof,
to give a compound of the formula:

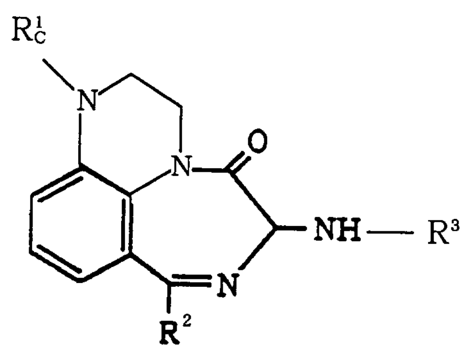

wherein $R^2$,$R^3$ and $R_c^1$ are each as defined above,
or a salt thereof.

**9.** A compound of the formula:

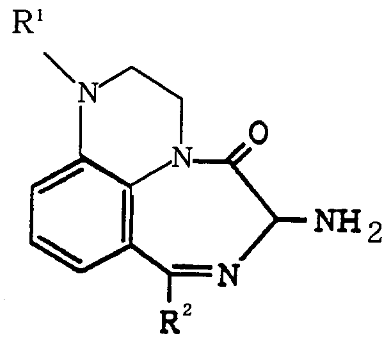

wherein $R^1$ is hydrogen,carboxy,protected carboxy, carboxy (lower) alkyl, protected carboxy (lower) alkyl, carboxy (lower) alkanoyl, protected carboxy (lower) alkanoyl, carboxy (lower) alkenoyl, protected carboxy (lower) alkenoyl, heterocycliccarbonyl (lower) alkyl which may have suitable substituent (s), or heterocycliccarbonyl (lower) alkanoyl which may have suitable substituent (s),

$R^2$ is aryl which may have suitable substituent (s), and a salt thereof.

**10.** A pharmaceutical composition comprising the compound or the pharmaceutically acceptable salt thereof as recited in claim 1, and a pharmaceutically acceptable carrier.

**11.** Use of the compound or the pharmaceutically acceptable salt thereof as recited in claim 1 as a cholecystokinin antagonist.

**12.** A method for preventing or treating diseases caused by cholecystokinin, which comprises administering the compound or the pharmaceutically acceptable salt thereof as recited in claim 1 to human beings or animals.

## INTERNATIONAL SEARCH REPORT

International application No.
PCT/JP93/01601

A. CLASSIFICATION OF SUBJECT MATTER

Int. $Cl^5$ C07D487/06, A61K31/55

According to International Patent Classification (IPC) or to both national classification and IPC

B. FIELDS SEARCHED

Minimum documentation searched (classification system followed by classification symbols)

Int. $Cl^5$ C07D487/06, A61K31/55

Documentation searched other than minimum documentation to the extent that such documents are included in the fields searched

Electronic data base consulted during the international search (name of data base and, where practicable, search terms used)

CAS ONLINE

C. DOCUMENTS CONSIDERED TO BE RELEVANT

| Category* | Citation of document, with indication, where appropriate, of the relevant passages | Relevant to claim No. |
|---|---|---|
| X | WO, A, 9203438 (Fujisawa Pharmaceutical Co., Ltd.), March 5, 1992 (05. 03. 92) | 1-10 |
| A | JP, A, 46-4827 (A. H. Robins Co., Inc.), November 19, 1971 (19. 11. 71), (Family: none) | 1-9 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| January 6, 1994 (06. 01. 94) | January 25, 1994 (25. 01. 94) |
| Name and mailing address of the ISA/<br>Japanese Patent Office<br>Facsimile No. | Authorized officer<br><br>Telephone No. |

**INTERNATIONAL SEARCH REPORT**

International application No.
PCT/JP93/01601

**Box I Observations where certain claims were found unsearchable (Continuation of item 1 of first sheet)**

This international search report has not been established in respect of certain claims under Article 17(2)(a) for the following reasons:

1. [X] Claims Nos.: 11, 12
because they relate to subject matter not required to be searched by this Authority, namely:
Claims 11 and 12 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.